(19) Europäisches Patentamt / European Patent Office / Office européen des brevets

(11) **EP 3 221 336 B1**

(12) **EUROPEAN PATENT SPECIFICATION**

(45) Date of publication and mention
of the grant of the patent:
**22.04.2020 Bulletin 2020/17**

(21) Application number: **15798047.5**

(22) Date of filing: **19.11.2015**

(51) Int Cl.:
*C07K 7/08* (2006.01)　　　*C07K 7/06* (2006.01)

(86) International application number:
**PCT/EP2015/077127**

(87) International publication number:
**WO 2016/079250 (26.05.2016 Gazette 2016/21)**

(54) **INFLUENZA VIRUS BINDING PEPTIDES**

INFLUENZAVIRUS-BINDENDE PEPTIDE

PEPTIDES DE LIAISON DU VIRUS DE LA GRIPPE

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **19.11.2014 EP 14193922**

(43) Date of publication of application:
**27.09.2017 Bulletin 2017/39**

(73) Proprietor: **Universität Potsdam
14469 Potsdam (DE)**

(72) Inventors:
• **STÖCKLEIN, Walter F.M.
14542 Werder (Havel) (DE)**
• **EHRENTREICH-FÖRSTER, Eva
14558 Nuthetal (DE)**
• **BIER, Frank F.
14482 Potsdam (DE)**
• **MEMCZAK, Henry
14476 Potsdam (DE)**
• **LAUSTER, Daniel
10439 Berlin (DE)**
• **HERRMANN, Andreas
14467 Potsdam (DE)**

(74) Representative: **Andresen, Heiko
Hansepatent Patentanwälte
Andresen Scholz PartG mbB
Poststraße 33
20354 Hamburg (DE)**

(56) References cited:
**WO-A2-2007/038172　　　WO-A2-2011/146514**

• **ROBBINS P B ET AL: "Peptide delivery to tissues
via reversibly linked protein transduction
sequences", BIOTECHNIQUES 2002 EATON
PUBLISHING COMPANY US, vol. 33, no. 1, 2002,
pages 190-194, XP001526573,**
• **ARYA SUNIL K ET AL: "Label free biosensor for
sensitive human influenza virus hemagglutinin
specific antibody detection using coiled-coil
peptide modified microelectrode array based
platform", SENSORS AND ACTUATORS B:
CHEMICAL: INTERNATIONAL JOURNAL
DEVOTED TO RESEARCH AND DEVELOPMENT
OF PHYSICAL AND CHEMICAL TRANSDUCERS,
vol. 194, 24 December 2013 (2013-12-24), pages
127-133, XP028609190, ISSN: 0925-4005, DOI:
10.1016/J.SNB.2013.12.066**

SEQUENCE LISTING

<110> Fraunhofer Gesellschaft zur Foerderung der angewandten Forschung e.V.

<120> Influenza virus binding peptides

<130> P2014,1193 EP E

<160> 104

<170> PatentIn version 3.5

<210> 1
<211> 8
<212> PRT
<213> Mouse

<400> 1

```
Phe Tyr Asp Tyr Asp Val Phe Tyr
1               5
```

<210> 2
<211> 8
<212> PRT
<213> artificial

<220>
<223> Artificial sequence

<220>
<221> X
<222> (1)..(1)
<223> selected from Ala, Asn, Asp, Arg, Cys, Gln, Glu, Gly, His, Ile, Leu, Lys, Met, Phe, Pro, Ser, Thr, Trp, Tyr and Val

<220>
<221> X
<222> (2)..(2)
<223> selected from Ala, Asn, Asp, Arg, Cys, Gln, Glu, Gly, His, Ile, Leu, Lys, Met, Phe, Pro, Ser, Thr, Trp, Tyr and Val

<220>
<221> X
<222> (3)..(3)
<223> selected from Ala, Asn, Asp, Arg, Cys, Gln, Glu, Gly, His, Ile, Leu, Lys, Met, Phe, Pro, Ser, Thr, Trp, Tyr and Val

<220>
<221> X
<222> (4)..(4)
<223> selected from Ala, Asn, Asp, Arg, Cys, Gln, Glu, Gly, His, Ile, Leu, Lys, Met, Phe, Pro, Ser, Thr, Trp, Tyr and Val

<220>
<221> X
<222> (6)..(6)
<223> selected from Ala, Asn, Asp, Arg, Cys, Gln, Glu, Gly, His, Ile, Leu, Lys, Met, Phe, Pro, Ser, Thr, Trp, Tyr and Val

<220>

<221> X
<222> (7)..(7)
<223> selected from Ala, Asn, Asp, Arg, Cys, Gln, Glu, Gly, His, Ile, Leu, Lys, Met, Phe, Pro, Ser, Thr, Trp, Tyr and Val

<220>
<221> X
<222> (8)..(8)
<223> selected from Ala, Asn, Asp, Arg, Cys, Gln, Glu, Gly, His, Ile, Leu, Lys, Met, Phe, Pro, Ser, Thr, Trp, Tyr and Val

<400> 2

```
Xaa Xaa Xaa Xaa Asp Xaa Xaa Xaa
1               5
```

<210> 3
<211> 8
<212> PRT
<213> artificial

<220>
<223> Artificial sequence

<220>
<221> X
<222> (1)..(1)
<223> selected from Ala, Asn, Asp, Arg, Cys, Gln, Glu, Gly, His, Ile, Leu, Lys, Met, Phe, Pro, Ser, Thr, Trp, Tyr and Val

<220>
<221> X
<222> (2)..(2)
<223> selected from Ala, Asn, Asp, Arg, Cys, Gln, Glu, Gly, His, Ile, Leu, Lys, Met, Phe, Pro, Ser, Thr, Trp, Tyr and Val

<220>
<221> X
<222> (3)..(3)
<223> selected from Ala, Asn, Asp, Arg, Cys, Gln, Glu, Gly, His, Ile, Leu, Lys, Met, Phe, Pro, Ser, Thr, Trp, Tyr and Val

<220>
<221> X
<222> (6)..(6)
<223> selected from Ala, Asn, Asp, Arg, Cys, Gln, Glu, Gly, His, Ile, Leu, Lys, Met, Phe, Pro, Ser, Thr, Trp, Tyr and Val

<220>
<221> X
<222> (7)..(7)
<223> selected from Ala, Asn, Asp, Arg, Cys, Gln, Glu, Gly, His, Ile, Leu, Lys, Met, Phe, Pro, Ser, Thr, Trp, Tyr and Val

<220>
<221> X
<222> (8)..(8)
<223> selected from Ala, Asn, Asp, Arg, Cys, Gln, Glu, Gly, His, Ile, Leu, Lys, Met, Phe, Pro, Ser, Thr, Trp, Tyr and Val

<400> 3

```
Xaa Xaa Xaa Tyr Asp Xaa Xaa Xaa
1               5
```

<210> 4

<211> 8
<212> PRT
<213> artificial

<220>
<223> Artificial sequence

<220>
<221> X
<222> (1)..(1)
<223> selected from Ala, Asn, Asp, Arg, Cys, Gln, Glu, Gly, His, Ile, Leu, Lys, Met, Phe, Pro, Ser, Thr, Trp, Tyr and Val

<220>
<221> X
<222> (2)..(2)
<223> selected from Ala, Asn, Asp, Arg, Cys, Gln, Glu, Gly, His, Ile, Leu, Lys, Met, Phe, Pro, Ser, Thr, Trp, Tyr and Val

<220>
<221> X
<222> (3)..(3)
<223> selected from Ala, Asn, Asp, Arg, Cys, Gln, Glu, Gly, His, Ile, Leu, Lys, Met, Phe, Pro, Ser, Thr, Trp, Tyr and Val

<220>
<221> X
<222> (7)..(7)
<223> selected from Ala, Asn, Asp, Arg, Cys, Gln, Glu, Gly, His, Ile, Leu, Lys, Met, Phe, Pro, Ser, Thr, Trp, Tyr and Val

<220>
<221> X
<222> (8)..(8)
<223> selected from Ala, Asn, Asp, Arg, Cys, Gln, Glu, Gly, His, Ile, Leu, Lys, Met, Phe, Pro, Ser, Thr, Trp, Tyr and Val

<400> 4

```
Xaa Xaa Xaa Tyr Asp Val Xaa Xaa
1               5
```

<210> 5
<211> 8
<212> PRT
<213> artificial

<220>
<223> Artificial sequence

<220>
<221> X
<222> (1)..(1)
<223> selected from Ala, Asn, Asp, Arg, Cys, Gln, Glu, Gly, His, Ile, Leu, Lys, Met, Phe, Pro, Ser, Thr, Trp, Tyr and Val

<220>
<221> X
<222> (3)..(3)
<223> selected from Ala, Asn, Asp, Arg, Cys, Gln, Glu, Gly, His, Ile, Leu, Lys, Met, Phe, Pro, Ser, Thr, Trp, Tyr and Val

<220>
<221> X
<222> (6)..(6)

<223> selected from Ala, Asn, Asp, Arg, Cys, Gln, Glu, Gly, His, Ile, Leu, Lys, Met, Phe, Pro, Ser, Thr, Trp, Tyr and Val

<220>
<221> X
<222> (7)..(7)
<223> selected from Ala, Asn, Asp, Arg, Cys, Gln, Glu, Gly, His, Ile, Leu, Lys, Met, Phe, Pro, Ser, Thr, Trp, Tyr and Val

<220>
<221> X
<222> (8)..(8)
<223> selected from Ala, Asn, Asp, Arg, Cys, Gln, Glu, Gly, His, Ile, Leu, Lys, Met, Phe, Pro, Ser, Thr, Trp, Tyr and Val

<400> 5

```
        Xaa Tyr Xaa Tyr Asp Xaa Xaa Xaa
        1               5
```

<210> 6
<211> 8
<212> PRT
<213> artificial

<220>
<223> Artificial sequence

<220>
<221> X
<222> (1)..(1)
<223> selected from Ala, Asn, Asp, Arg, Cys, Gln, Glu, Gly, His, Ile, Leu, Lys, Met, Phe, Pro, Ser, Thr, Trp, Tyr and Val

<220>
<221> X
<222> (3)..(3)
<223> selected from Ala, Asn, Asp, Arg, Cys, Gln, Glu, Gly, His, Ile, Leu, Lys, Met, Phe, Pro, Ser, Thr, Trp, Tyr and Val

<220>
<221> X
<222> (7)..(7)
<223> selected from Ala, Asn, Asp, Arg, Cys, Gln, Glu, Gly, His, Ile, Leu, Lys, Met, Phe, Pro, Ser, Thr, Trp, Tyr and Val

<220>
<221> X
<222> (8)..(8)
<223> selected from Ala, Asn, Asp, Arg, Cys, Gln, Glu, Gly, His, Ile, Leu, Lys, Met, Phe, Pro, Ser, Thr, Trp, Tyr and Val

<400> 6

```
        Xaa Tyr Xaa Tyr Asp Val Xaa Xaa
        1               5
```

<210> 7
<211> 8
<212> PRT
<213> artificial

<220>
<223> Artificial sequence

<220>
<221> X
<222> (1)..(1)
<223> selected from Ala, Asn, Asp, Arg, Cys, Gln, Glu, Gly, His, Ile, Leu, Lys, Met, Phe, Pro, Ser, Thr, Trp, Tyr and Val

<220>
<221> X
<222> (3)..(3)
<223> selected from Ala, Asn, Asp, Arg, Cys, Gln, Glu, Gly, His, Ile, Leu, Lys, Met, Phe, Pro, Ser, Thr, Trp, Tyr and Val

<220>
<221> X
<222> (6)..(6)
<223> selected from Ala, Asn, Asp, Arg, Cys, Gln, Glu, Gly, His, Ile, Leu, Lys, Met, Phe, Pro, Ser, Thr, Trp, Tyr and Val

<220>
<221> X
<222> (8)..(8)
<223> selected from Ala, Asn, Asp, Arg, Cys, Gln, Glu, Gly, His, Ile, Leu, Lys, Met, Phe, Pro, Ser, Thr, Trp, Tyr and Val

<400> 7

```
Xaa Tyr Xaa Tyr Asp Xaa Phe Xaa
1               5
```

<210> 8
<211> 8
<212> PRT
<213> artificial

<220>
<223> Artificial sequence

<220>
<221> x
<222> (1)..(1)
<223> selected from Ala, Asn, Asp, Arg, Cys, Gln, Glu, Gly, His, Ile, Leu, Lys, Met, Phe, Pro, Ser, Thr, Trp, Tyr and Val

<220>
<221> x
<222> (2)..(2)
<223> selected from Ala, Asn, Asp, Arg, Cys, Gln, Glu, Gly, His, Ile, Leu, Lys, Met, Phe, Pro, Ser, Thr, Trp, Tyr and Val

<220>
<221> x
<222> (3)..(3)
<223> selected from Ala, Asn, Asp, Arg, Cys, Gln, Glu, Gly, His, Ile, Leu, Lys, Met, Phe, Pro, Ser, Thr, Trp, Tyr and Val

<220>
<221> x
<222> (8)..(8)
<223> selected from Ala, Asn, Asp, Arg, Cys, Gln, Glu, Gly, His, Ile, Leu, Lys, Met, Phe, Pro, Ser, Thr, Trp, Tyr and Val

<400> 8

```
Xaa Xaa Xaa Tyr Asp Val Phe Xaa
1               5
```

<210> 9
<211> 8
<212> PRT
<213> artificial

<220>
<223> Artificial sequence

<220>
<221> X
<222> (3)..(3)
<223> selected from Ala, Asn, Asp, Arg, Cys, Gln, Glu, Gly, His, Ile, Leu, Lys, Met, Phe, Pro, Ser, Thr, Trp, Tyr and Val

<220>
<221> X
<222> (7)..(7)
<223> selected from Ala, Asn, Asp, Arg, Cys, Gln, Glu, Gly, His, Ile, Leu, Lys, Met, Phe, Pro, Ser, Thr, Trp, Tyr and Val

<220>
<221> X
<222> (8)..(8)
<223> selected from Ala, Asn, Asp, Arg, Cys, Gln, Glu, Gly, His, Ile, Leu, Lys, Met, Phe, Pro, Ser, Thr, Trp, Tyr and Val

<400> 9

```
Phe Tyr Xaa Tyr Asp Val Xaa Xaa
1               5
```

<210> 10
<211> 8
<212> PRT
<213> artificial

<220>
<223> Artificial sequence

<220>
<221> X
<222> (1)..(4)
<223> selected from Ala, Asn, Asp, Arg, Cys, Gln, Glu, Gly, His, Ile, Leu, Lys, Met, Phe, Pro, Ser, Thr, Trp, Tyr and Val

<220>
<221> X
<222> (6)..(8)
<223> selected from Ala, Asn, Asp, Arg, Cys, Gln, Glu, Gly, His, Ile, Leu, Lys, Met, Phe, Pro, Ser, Thr, Trp, Tyr and Val

<400> 10

```
Xaa Tyr Xaa Tyr Asp Val Phe Xaa
1               5
```

<210> 11
<211> 8
<212> PRT
<213> artificial

<220>
<223> Artificial sequence

<220>
<221> X
<222> (2)..(2)
<223> selected from Ala, Asn, Asp, Arg, Cys, Gln, Glu, Gly, His, Ile, Leu, Lys, Met, Phe, Pro, Ser, Thr, Trp, Tyr and Val

<220>
<221> X
<222> (3)..(3)
<223> selected from Ala, Asn, Asp, Arg, Cys, Gln, Glu, Gly, His, Ile, Leu, Lys, Met, Phe, Pro, Ser, Thr, Trp, Tyr and Val

<220>
<221> X
<222> (8)..(8)
<223> selected from Ala, Asn, Asp, Arg, Cys, Gln, Glu, Gly, His, Ile, Leu, Lys, Met, Phe, Pro, Ser, Thr, Trp, Tyr and Val

<400> 11

```
                    Phe Xaa Xaa Tyr Asp Val Phe Xaa
                    1                   5
```

<210> 12
<211> 8
<212> PRT
<213> artificial

<220>
<223> Artificial sequence

<220>
<221> X
<222> (3)..(3)
<223> selected from Ala, Asn, Asp, Arg, Cys, Gln, Glu, Gly, His, Ile, Leu, Lys, Met, Phe, Pro, Ser, Thr, Trp, Tyr and Val

<220>
<221> X
<222> (6)..(6)
<223> selected from Ala, Asn, Asp, Arg, Cys, Gln, Glu, Gly, His, Ile, Leu, Lys, Met, Phe, Pro, Ser, Thr, Trp, Tyr and Val

<220>
<221> X
<222> (8)..(8)
<223> selected from Ala, Asn, Asp, Arg, Cys, Gln, Glu, Gly, His, Ile, Leu, Lys, Met, Phe, Pro, Ser, Thr, Trp, Tyr and Val

<400> 12

```
                    Phe Tyr Xaa Tyr Asp Xaa Phe Xaa
                    1                   5
```

<210> 13
<211> 8
<212> PRT
<213> artificial

<220>
<223> Artificial sequence

<220>
<221> X

<222> (3)..(3)
<223> selected from Ala, Asn, Asp, Arg, Cys, Gln, Glu, Gly, His, Ile, Leu, Lys, Met, Phe, Pro, Ser, Thr, Trp, Tyr and Val

<220>
<221> X
<222> (8)..(8)
<223> selected from Ala, Asn, Asp, Arg, Cys, Gln, Glu, Gly, His, Ile, Leu, Lys, Met, Phe, Pro, Ser, Thr, Trp, Tyr and Val

<400> 13

```
                    Phe Tyr Xaa Tyr Asp Val Phe Xaa
                    1               5
```

<210> 14
<211> 8
<212> PRT
<213> artificial

<220>
<223> Artificial sequence

<220>
<221> X
<222> (3)..(3)
<223> selected from Ala, Asn, Asp, Arg, Cys, Gln, Glu, Gly, His, Ile, Leu, Lys, Met, Phe, Pro, Ser, Thr, Trp, Tyr and Val

<220>
<221> X
<222> (7)..(7)
<223> selected from Ala, Asn, Asp, Arg, Cys, Gln, Glu, Gly, His, Ile, Leu, Lys, Met, Phe, Pro, Ser, Thr, Trp, Tyr and Val

<400> 14

```
                    Phe Tyr Xaa Tyr Asp Val Xaa Tyr
                    1               5
```

<210> 15
<211> 8
<212> PRT
<213> artificial

<220>
<223> Artificial sequence

<220>
<221> X
<222> (3)..(3)
<223> selected from Ala, Asn, Asp, Arg, Cys, Gln, Glu, Gly, His, Ile, Leu, Lys, Met, Phe, Pro, Ser, Thr, Trp, Tyr and Val

<220>
<221> X
<222> (6)..(6)
<223> selected from Ala, Asn, Asp, Arg, Cys, Gln, Glu, Gly, His, Ile, Leu, Lys, Met, Phe, Pro, Ser, Thr, Trp, Tyr and Val

<400> 15

```
                    Phe Tyr Xaa Tyr Asp Xaa Phe Tyr
                    1               5
```

<210> 16
<211> 8
<212> PRT
<213> artificial

<220>
<223> Artificial sequence

<220>
<221> X
<222> (1)..(1)
<223> selected from Ala, Asn, Asp, Arg, Cys, Gln, Glu, Gly, His, Ile, Leu, Lys, Met, Phe, Pro, Ser, Thr, Trp, Tyr and Val

<400> 16

```
                    Xaa Tyr Asp Tyr Asp Val Phe Tyr
                    1               5
```

<210> 17
<211> 8
<212> PRT
<213> artificial

<220>
<223> Artificial sequence

<220>
<221> X
<222> (2)..(2)
<223> selected from Ala, Asn, Asp, Arg, Cys, Gln, Glu, Gly, His, Ile, Leu, Lys, Met, Phe, Pro, Ser, Thr, Trp, Tyr and Val

<400> 17

```
                    Phe Xaa Asp Tyr Asp Val Phe Tyr
                    1               5
```

<210> 18
<211> 8
<212> PRT
<213> artificial

<220>
<223> Artificial sequence

<220>
<221> X
<222> (3)..(3)
<223> selected from Ala, Asn, Asp, Arg, Cys, Gln, Glu, Gly, His, Ile, Leu, Lys, Met, Phe, Pro, Ser, Thr, Trp, Tyr and Val

<400> 18

```
                    Phe Tyr Xaa Tyr Asp Val Phe Tyr
                    1               5
```

<210> 19
<211> 8
<212> PRT
<213> artificial

<220>
<223> Artificial sequence

<220>
<221> X
<222> (4)..(4)
<223> selected from Ala, Asn, Asp, Arg, Cys, Gln, Glu, Gly, His, Ile, Leu, Lys, Met, Phe, Pro, Ser, Thr, Trp, Tyr and Val

<400> 19

```
                    Phe Tyr Asp Xaa Asp Val Phe Tyr
                    1               5
```

<210> 20
<211> 8
<212> PRT
<213> artificial

<220>
<223> Artificial sequence

<220>
<221> X
<222> (6)..(6)
<223> selected from Ala, Asn, Asp, Arg, Cys, Gln, Glu, Gly, His, Ile, Leu, Lys, Met, Phe, Pro, Ser, Thr, Trp, Tyr and Val

<400> 20

```
                    Phe Tyr Asp Tyr Asp Xaa Phe Tyr
                    1               5
```

<210> 21
<211> 8
<212> PRT
<213> artificial

<220>
<223> Artificial sequence

<220>
<221> X
<222> (7)..(7)
<223> selected from Ala, Asn, Asp, Arg, Cys, Gln, Glu, Gly, His, Ile, Leu, Lys, Met, Phe, Pro, Ser, Thr, Trp, Tyr and Val

<400> 21

```
                    Phe Tyr Asp Tyr Asp Val Xaa Tyr
                    1               5
```

<210> 22
<211> 8
<212> PRT
<213> artificial

<220>
<223> Artificial sequence

<220>

<221> X
<222> (8)..(8)
<223> selected from Ala, Asn, Asp, Arg, Cys, Gln, Glu, Gly, His, Ile, Leu, Lys, Met, Phe, Pro, Ser, Thr, Trp, Tyr and Val

<400> 22

```
                              Phe Tyr Asp Tyr Asp Val Phe Xaa
                              1               5
```

<210> 23
<211> 8
<212> PRT
<213> artificial

<220>
<223> Artificial sequence

<400> 23

```
                              Phe Tyr Tyr Tyr Asp Val Phe Tyr
                              1               5
```

<210> 24
<211> 8
<212> PRT
<213> artificial

<220>
<223> Artificial sequence

<400> 24

```
                              Phe Tyr Asp Pro Asp Val Phe Tyr
                              1               5
```

<210> 25
<211> 8
<212> PRT
<213> artificial

<220>
<223> Artificial sequence

<400> 25

```
                              Phe Tyr Asp Tyr Asp Pro Phe Tyr
                              1               5
```

<210> 26
<211> 8
<212> PRT
<213> artificial

<220>
<223> Artificial sequence

<400> 26

```
Phe Tyr Asp Tyr Asp Gln Phe Tyr
1                   5
```

<210> 27
<211> 8
<212> PRT
<213> artificial

<220>
<223> Artificial sequence

<400> 27

```
Phe Tyr Asp Tyr Asp Val Phe Phe
1                   5
```

<210> 28
<211> 8
<212> PRT
<213> artificial

<220>
<223> Artificial sequence

<400> 28

```
Phe Tyr Gly Tyr Asp Val Phe Phe
1                   5
```

<210> 29
<211> 8
<212> PRT
<213> artificial

<220>
<223> Artificial sequence

<400> 29

```
Phe Tyr Tyr Tyr Asp Val Phe Phe
1                   5
```

<210> 30
<211> 8
<212> PRT
<213> artificial

<220>
<223> Artificial sequence

<400> 30

```
Phe Tyr Gly Tyr Asp Gln Phe Tyr
1                   5
```

<210> 31
<211> 8
<212> PRT

<213> artificial

<220>
<223> Artificial sequence

<400> 31

```
                              Phe Tyr Tyr Tyr Asp Gln Phe Tyr
                              1               5
```

<210> 32
<211> 8
<212> PRT
<213> artificial

<220>
<223> Artificial sequence

<400> 32

```
                              Leu Tyr Gly Tyr Asp Val Phe Phe
                              1               5
```

<210> 33
<211> 8
<212> PRT
<213> artificial

<220>
<223> Artificial sequence

<400> 33

```
                              Phe Tyr Gly Tyr Asp Val Ala Phe
                              1               5
```

<210> 34
<211> 8
<212> PRT
<213> artificial

<220>
<223> Artificial sequence

<400> 34

```
                              Phe Leu Gly Tyr Asp Val Phe Phe
                              1               5
```

<210> 35
<211> 8
<212> PRT
<213> artificial

<220>
<223> Artificial sequence

<400> 35

```
His Tyr Asp Tyr Asp Val Phe Tyr
1               5
```

<210> 36
<211> 8
<212> PRT
<213> artificial

<220>
<223> Artificial sequence

<400> 36

```
Ile Tyr Asp Tyr Asp Val Phe Tyr
1               5
```

<210> 37
<211> 8
<212> PRT
<213> artificial

<220>
<223> Artificial sequence

<400> 37

```
Trp Tyr Asp Tyr Asp Val Phe Tyr
1               5
```

<210> 38
<211> 8
<212> PRT
<213> artificial

<220>
<223> Artificial sequence

<400> 38

```
Asp Tyr Asp Tyr Asp Val Phe Tyr
1               5
```

<210> 39
<211> 8
<212> PRT
<213> artificial

<220>
<223> Artificial sequence

<400> 39

```
Glu Tyr Asp Tyr Asp Val Phe Tyr
1               5
```

<210> 40
<211> 8
<212> PRT

<213> artificial

<220>
<223> Artificial sequence

<400> 40

```
                              Gly Tyr Asp Tyr Asp Val Phe Tyr
                              1               5
```

<210> 41
<211> 8
<212> PRT
<213> artificial

<220>
<223> Artificial sequence

<400> 41

```
                              Ile Tyr Asp Tyr Asp Val Phe Tyr
                              1               5
```

<210> 42
<211> 8
<212> PRT
<213> artificial

<220>
<223> Artificial sequence

<400> 42

```
                              Lys Tyr Asp Tyr Asp Val Phe Tyr
                              1               5
```

<210> 43
<211> 8
<212> PRT
<213> artificial

<220>
<223> Artificial sequence

<400> 43

```
                              Leu Tyr Asp Tyr Asp Val Phe Tyr
                              1               5
```

<210> 44
<211> 8
<212> PRT
<213> artificial

<220>
<223> Artificial sequence

<400> 44

```
                          Asn Tyr Asp Tyr Asp Val Phe Tyr
                          1               5
```

<210> 45
<211> 8
<212> PRT
<213> artificial

<220>
<223> Artificial sequence

<400> 45

```
                          Pro Tyr Asp Tyr Asp Val Phe Tyr
                          1               5
```

<210> 46
<211> 8
<212> PRT
<213> artificial

<220>
<223> Artificial sequence

<400> 46

```
                          Arg Tyr Asp Tyr Asp Val Phe Tyr
                          1               5
```

<210> 47
<211> 8
<212> PRT
<213> artificial

<220>
<223> Artificial sequence

<400> 47

```
                          Phe Pro Asp Tyr Asp Val Phe Tyr
                          1               5
```

<210> 48
<211> 8
<212> PRT
<213> artificial

<220>
<223> Artificial sequence

<400> 48

```
                          Phe Gln Asp Tyr Asp Val Phe Tyr
                          1               5
```

<210> 49
<211> 8
<212> PRT

<213> artificial

<220>
<223> Artificial sequence

<400> 49

```
                              Phe Arg Asp Tyr Asp Val Phe Tyr
                              1               5
```

<210> 50
<211> 8
<212> PRT
<213> artificial

<220>
<223> Artificial sequence

<400> 50

```
                              Phe Cys Asp Tyr Asp Val Phe Tyr
                              1               5
```

<210> 51
<211> 8
<212> PRT
<213> artificial

<220>
<223> Artificial sequence

<400> 51

```
                              Phe Asp Asp Tyr Asp Val Phe Tyr
                              1               5
```

<210> 52
<211> 8
<212> PRT
<213> artificial

<220>
<223> Artificial sequence

<400> 52

```
                              Phe Glu Asp Tyr Asp Val Phe Tyr
                              1               5
```

<210> 53
<211> 8
<212> PRT
<213> artificial

<220>
<223> Artificial sequence

<400> 53

```
                           Phe Gly Asp Tyr Asp Val Phe Tyr
                           1               5
```

<210> 54
<211> 8
<212> PRT
<213> artificial

<220>
<223> Artificial sequence

<400> 54

```
                           Phe Ile Asp Tyr Asp Val Phe Tyr
                           1               5
```

<210> 55
<211> 8
<212> PRT
<213> artificial

<220>
<223> Artificial sequence

<400> 55

```
                           Phe Lys Asp Tyr Asp Val Phe Tyr
                           1               5
```

<210> 56
<211> 8
<212> PRT
<213> artificial

<220>
<223> Artificial sequence

<400> 56

```
                           Phe Leu Asp Tyr Asp Val Phe Tyr
                           1               5
```

<210> 57
<211> 8
<212> PRT
<213> artificial

<220>
<223> Artificial sequence

<400> 57

```
                           Phe Met Asp Tyr Asp Val Phe Tyr
                           1               5
```

<210> 58
<211> 8
<212> PRT

<213> artificial

<220>
<223> Artificial sequence

<400> 58

```
                        Phe Ser Asp Tyr Asp Val Phe Tyr
                        1               5
```

<210> 59
<211> 8
<212> PRT
<213> artificial

<220>
<223> Artificial sequence

<400> 59

```
                        Phe Tyr Phe Tyr Asp Val Phe Tyr
                        1               5
```

<210> 60
<211> 8
<212> PRT
<213> artificial

<220>
<223> Artificial sequence

<400> 60

```
                        Phe Tyr Gly Tyr Asp Val Phe Tyr
                        1               5
```

<210> 61
<211> 8
<212> PRT
<213> artificial

<220>
<223> Artificial sequence

<400> 61

```
                        Phe Tyr Gln Tyr Asp Val Phe Tyr
                        1               5
```

<210> 62
<211> 8
<212> PRT
<213> artificial

<220>
<223> Artificial sequence

<400> 62

```
Phe Tyr Thr Tyr Asp Val Phe Tyr
1               5
```

<210> 63
<211> 8
<212> PRT
<213> artificial

<220>
<223> Artificial sequence

<400> 63

```
Phe Tyr Trp Tyr Asp Val Phe Tyr
1               5
```

<210> 64
<211> 8
<212> PRT
<213> artificial

<220>
<223> Artificial sequence

<400> 64

```
Phe Tyr Leu Tyr Asp Val Phe Tyr
1               5
```

<210> 65
<211> 8
<212> PRT
<213> artificial

<220>
<223> Artificial sequence

<400> 65

```
Phe Tyr Asp Ala Asp Val Phe Tyr
1               5
```

<210> 66
<211> 8
<212> PRT
<213> artificial

<220>
<223> Artificial sequence

<400> 66

```
Phe Tyr Asp Cys Asp Val Phe Tyr
1               5
```

<210> 67
<211> 8
<212> PRT

<213> artificial

<220>
<223> Artificial sequence

<400> 67

```
                              Phe Tyr Asp Asp Asp Val Phe Tyr
                              1               5
```

<210> 68
<211> 8
<212> PRT
<213> artificial

<220>
<223> Artificial sequence

<400> 68

```
                              Phe Tyr Asp Glu Asp Val Phe Tyr
                              1               5
```

<210> 69
<211> 8
<212> PRT
<213> artificial

<220>
<223> Artificial sequence

<400> 69

```
                              Phe Tyr Asp Leu Asp Val Phe Tyr
                              1               5
```

<210> 70
<211> 8
<212> PRT
<213> artificial

<220>
<223> Artificial sequence

<400> 70

```
                              Phe Tyr Asp Met Asp Val Phe Tyr
                              1               5
```

<210> 71
<211> 8
<212> PRT
<213> artificial

<220>
<223> Artificial sequence

<400> 71

```
                              Phe Tyr Asp Pro Asp Val Phe Tyr
                              1               5
```

<210> 72
<211> 8
<212> PRT
<213> artificial

<220>
<223> Artificial sequence

<400> 72

```
                              Phe Tyr Asp Val Asp Val Phe Tyr
                              1               5
```

<210> 73
<211> 8
<212> PRT
<213> artificial

<220>
<223> Artificial sequence

<400> 73

```
                              Phe Tyr Asp Thr Asp Val Phe Tyr
                              1               5
```

<210> 74
<211> 8
<212> PRT
<213> artificial

<220>
<223> Artificial sequence

<400> 74

```
                              Phe Tyr Asp Ile Asp Val Phe Tyr
                              1               5
```

<210> 75
<211> 8
<212> PRT
<213> artificial

<220>
<223> Artificial sequence

<400> 75

```
                              Phe Tyr Asp Tyr Asp Asp Phe Tyr
                              1               5
```

<210> 76
<211> 8
<212> PRT

<213> artificial

<220>
<223> Artificial sequence

<400> 76

```
Phe Tyr Asp Tyr Asp Arg Phe Tyr
1               5
```

<210> 77
<211> 8
<212> PRT
<213> artificial

<220>
<223> Artificial sequence

<400> 77

```
Phe Tyr Asp Tyr Asp Glu Phe Tyr
1               5
```

<210> 78
<211> 8
<212> PRT
<213> artificial

<220>
<223> Artificial sequence

<400> 78

```
Phe Tyr Asp Tyr Asp Val Asp Tyr
1               5
```

<210> 79
<211> 8
<212> PRT
<213> artificial

<220>
<223> Artificial sequence

<400> 79

```
Phe Tyr Asp Tyr Asp Val Pro Tyr
1               5
```

<210> 80
<211> 8
<212> PRT
<213> artificial

<220>
<223> Artificial sequence

<400> 80

```
                              Phe Tyr Asp Tyr Asp Val Cys Tyr
                              1               5
```

<210> 81
<211> 8
<212> PRT
<213> artificial

<220>
<223> Artificial sequence

<400> 81

```
                              Phe Tyr Asp Tyr Asp Val Glu Tyr
                              1               5
```

<210> 82
<211> 8
<212> PRT
<213> artificial

<220>
<223> Artificial sequence

<400> 82

```
                              Phe Tyr Asp Tyr Asp Val Ala Tyr
                              1               5
```

<210> 83
<211> 8
<212> PRT
<213> artificial

<220>
<223> Artificial sequence

<400> 83

```
                              Phe Tyr Asp Tyr Asp Val Lys Tyr
                              1               5
```

<210> 84
<211> 8
<212> PRT
<213> artificial

<220>
<223> Artificial sequence

<400> 84

```
                              Phe Tyr Asp Tyr Asp Val Gln Tyr
                              1               5
```

<210> 85
<211> 8
<212> PRT

<213> artificial

<220>
<223> Artificial sequence

<400> 85

```
                              Phe Tyr Asp Tyr Asp Val Gly Tyr
                              1                   5
```

<210> 86
<211> 8
<212> PRT
<213> artificial

<220>
<223> Artificial sequence

<400> 86

```
                              Phe Tyr Asp Tyr Asp Val Thr Tyr
                              1                   5
```

<210> 87
<211> 8
<212> PRT
<213> artificial

<220>
<223> Artificial sequence

<400> 87

```
                              Phe Tyr Asp Tyr Asp Val Phe Asn
                              1                   5
```

<210> 88
<211> 8
<212> PRT
<213> artificial

<220>
<223> Artificial sequence

<400> 88

```
                              Phe Tyr Asp Tyr Asp Val Phe Cys
                              1                   5
```

<210> 89
<211> 8
<212> PRT
<213> artificial

<220>
<223> Artificial sequence

<400> 89

```
Phe Tyr Asp Tyr Asp Val Phe Asp
1               5
```

<210> 90
<211> 8
<212> PRT
<213> artificial

<220>
<223> Artificial sequence

<400> 90

```
Phe Tyr Asp Tyr Asp Val Phe Glu
1               5
```

<210> 91
<211> 8
<212> PRT
<213> artificial

<220>
<223> Artificial sequence

<400> 91

```
Phe Tyr Asp Tyr Asp Val Phe Met
1               5
```

<210> 92
<211> 8
<212> PRT
<213> artificial

<220>
<223> Artificial sequence

<400> 92

```
Phe Tyr Asp Tyr Asp Val Phe Pro
1               5
```

<210> 93
<211> 8
<212> PRT
<213> artificial

<220>
<223> Artificial sequence

<400> 93

```
Phe Tyr Asp Tyr Asp Val Phe Leu
1               5
```

<210> 94
<211> 8
<212> PRT

<213> artificial

<220>
<223> Artificial sequence

<400> 94

```
                           Phe Tyr Asp Tyr Asp Val Phe Thr
                           1               5
```

<210> 95
<211> 8
<212> PRT
<213> artificial

<220>
<223> Artificial sequence

<400> 95

```
                           Phe Tyr Asp Tyr Asp Val Phe Trp
                           1               5
```

<210> 96
<211> 8
<212> PRT
<213> artificial

<220>
<223> Artificial sequence

<400> 96

```
                           Phe Tyr Asp Tyr Asp Val Phe Ser
                           1               5
```

<210> 97
<211> 8
<212> PRT
<213> artificial

<220>
<223> Artificial sequence

<400> 97

```
                           Phe Tyr Gly Tyr Asp Val Lys Phe
                           1               5
```

<210> 98
<211> 8
<212> PRT
<213> artificial

<220>
<223> Artificial sequence

<400> 98

```
Phe Tyr Gly Tyr Asp Val Gln Phe
1               5
```

<210> 99
<211> 20
<212> PRT
<213> artificial

<220>
<223> Artificial sequence

<400> 99

```
Arg Arg Lys Lys Ala Ala Val Ala Leu Leu Pro Ala Val Leu Leu Ala
1               5               10              15

Leu Leu Ala Pro
              20
```

<210> 100
<211> 15
<212> PRT
<213> artificial

<220>
<223> Artificial sequence

<400> 100

```
Ala Arg Leu Pro Arg Thr Met Val His Pro Lys Pro Ala Gln Pro
1               5               10              15
```

<210> 101
<211> 12
<212> PRT
<213> artificial

<220>
<223> Artificial sequence

<400> 101

```
His Ala Trp Asp Pro Ile Pro Ala Arg Asp Pro Phe
1               5               10
```

<210> 102
<211> 7
<212> PRT
<213> artificial

<220>
<223> Artificial sequence

<400> 102

```
Asn Asp Phe Arg Ser Lys Thr
1               5
```

<210> 103
<211> 15
<212> PRT
<213> Mouse

<400> 103

        Ala Arg Asp Phe Tyr Asp Tyr Asp Val Phe Tyr Tyr Ala Met Asp

          1           5               10              15

<210> 104
<211> 8
<212> PRT
<213> artificial

<220>
<223> Artificial sequence

<400> 104

        Phe Tyr Asp Tyr Pro Val Phe Tyr
        1           5

**Description**

This patent application claims the priority of European patent application no. 14193922.3 filed November 19, 2014.

The present invention relates to peptides which have the ability to bind hemagglutinin found on the surface of influenza viruses. These peptides are of therapeutic and diagnostic applicability.

The influenza virus is an RNA virus of the orthomyxoviridae family. It is the causative agent of influenza, an infectious disease commonly known as "the flu" which spreads around the world in seasonal epidemics and occasional pandemics, with hundreds of thousands to millions of deaths yearly.

All influenza viruses are similar in structure, including a viral envelope containing two main types of glycoproteins, namely hemagglutinin (HA) and neuramidase (NA). These proteins are targets for anti-viral drugs and antibodies. Given the high mutation and recombination rate of influenza viruses, however, both proteins are frequently subject to genetic and antigenic changes which allow the viruses to escape from inhibitory agents and neutralizing antibodies. For the same reasons, vaccines can only temporarily control recurring epidemics of influenza. So far, 18 serotypes of influenza A virus hemagglutinin (H1 to H18) are known, three of which (H1, H2 and H3) have adapted to the human population.

One strategy of combatting influenza infection is to counteract antigenic changes by developing antibodies which bind to conserved regions of hemagglutinin. Such antibodies may exhibit broadened specificity and neutralization potency in diagnosis and treatment of influenza virus infections. However, the development, production and quality control of antibodies is expensive and time-consuming.

More recently, hemagglutinin-binding peptides have been developed as a more economical alternative to antibodies using phage display, lead structure optimization and bioinformatic tools (Rajik et al., Int. J. Biol. Sci. 2009, 5, 543-548; Matsubara et al., J. Med. Chem. 2009, 52, 4247-4256; Matsubara et al., J. Med. Chem. 2010, 53, 4441-4449; Wu et al., PloS One 2011, 6, e23058; Li et al., Peptides 2011, 32, 1518-1525; Lopez-Martinez et al., Plos One 2013, 8, e76876). Nevertheless, the majority of these peptides still has a relatively high molecular weight. Moreover, little is known about their binding site within hemagglutinin and, accordingly, about their antiviral activity.

Therefore, there is an unmet need for a low-molecular weight peptide which binds to different hemagglutinin serotypes with high affinity and broad specificity, and which has the ability to inhibit virus infection.

This problem is solved by the subject-matters according to the independent claims.

The present invention is based on the unique discovery of the inventors that the sequence information of a naturally evolved anti-HA antibody can be advantageously used and refined to provide highly specific and highly affine HA-binding peptides.

Based on sequence information and crystallographic data, the inventors were able to reduce the size and structural complexity of a monoclonal anti-hemagglutinin antibody (HC19) to a single truncated peptide sequence FYDYDVFY (SEQ ID NO:1 ) capable of binding to the receptor site of HA.

Subsequently, the inventors were able to develop sequence variations of SEQ ID NO:1 which outperform the naturally evolved peptide in terms of affinity and specificity.

In particular, the inventors realized the essentiality of the aspartic acid residue in position 5 of the peptide for the binding capacity to HA, whereas up to three of the amino acids in positions 1 to 4 and 6 to 8 can be rationally substituted by another amino acid in order to refine the affinity and specificity of the peptide.

Accordingly, as a reference, a peptide having from 8 to 40 amino acids, comprising a sequence $X^1$-$X^2$-$X^3$-$X^4$-Asp-$X^5$-$X^6$-$X^7$ (SEQ ID NO:2),
wherein $X^1$ to $X^7$ are selected from Ala, Asn, Asp, Arg, Cys, Gln, Glu, Gly, His, Ile, Leu, Lys, Met, Phe, Pro, Ser, Thr, Trp, Tyr and Val is disclosed.

Furthermore, as a reference, the SEQ ID NO:2 has at least 62.5% sequence identity and at most 87.5% sequence identity with the sequence Phe-Tyr-Asp-Tyr-Asp-Val-Phe-Tyr (SEQ ID NO:1) .

The percentage of sequence identity between two peptide sequences can for example be determined with the algorithm "ClustalW" (Thompson et al., Nucleic Acid Res., 1994, 22, 4673-4680). A suitable tool for determining the percentage of sequence identity of two peptide sequences is BLASTP (Altschul et al., Nucleic Acids Res. 1997, 25, 3389-3402). For the purpose of describing the present invention, amino acids may be interchangeably identified by their full denomination as well as their commonly used one-letter and three-letter code (see, for example, Berg et al. "Biochemistry", W. H. Freeman and Company, New York 2002).

In other terms, as a reference, four of $X^1$ to $X^7$ are selected from a group consisting of: $X^1$ is Phe, $X^2$ is Tyr, $X^3$ is Asp, $X^4$ is Tyr, $X^5$ is Val, $X^6$ is Phe and $X^7$ is Tyr, whereas three of $X^1$ to $X^7$ do not belong to this group.

Alternatively, as a reference, five of $X^1$ to $X^7$ are selected from the group consisting of: $X^1$ is Phe, $X^2$ is Tyr, $X^3$ is Asp, $X^4$ is Tyr, $X^5$ is Val, $X^6$ is Phe and $X^7$ is Tyr, whereas two of $X^1$ to $X^7$ do not belong to this group.

In a further alternative, as a reference, six of $X^1$ to $X^7$ are selected from the group consisting of: $X^1$ is Phe, $X^2$ is Tyr, $X^3$ is Asp, $X^4$ is Tyr, $X^5$ is Val, $X^6$ is Phe and $X^7$ is Tyr, whereas one of $X^1$ to $X^7$ does not belong to this group.

The peptide of the present invention can exhibit an increased affinity to influenza hemagglutinin and virus inhibition capability in comparison to its natural counterpart. The affinity between the peptide and influenza A virus hemagglutinin may be defined by the equilibrium dissociation constant $K_D$. For example, the $K_D$ between the peptide and influenza A virus hemagglutinin is lower than 59 $\mu$M, preferably lower than 40 $\mu$M, more preferably lower than 25 $\mu$M. Methods for determining the equilibrium dissociation constant $K_D$ are well-known in the art. A suitable method is also described in example 3 further below.

As used herein, the virus inhibition capability may be characterized by the peptide concentration required for 50% inhibition of viral binding to its host cell receptor ($IC_{50,RB}$) as for instance set out in example 4 further below. The $IC_{50,RB}$ of the peptide is preferably lower than 90 $\mu$M, more preferably lower than 50 $\mu$M, most preferred lower than 25 $\mu$M.

The virus inhibition capability may also be described in terms of the hemagglutination inhibition constant $K_i(HAI)$, which reflects the lowest peptide concentration required for achieving 100% hemagglutination inhibition. Preferably, the peptide has a $K_i(HAI)$ lower than 235 $\mu$M, more preferably lower than 100 $\mu$M, most preferred lower than 50 $\mu$M. Methods for determining the hemagglutination inhibition constant $K_i(HAI)$ are well-known in the art. One suitable method is described in example 5 further below.

The virus inhibition capability may also be measured in terms of the peptide concentration required for 50% inhibition of viral hemagglutination ($IC_{50,HAI}$). Preferably, the $IC_{50,HAI}$ is lower than 47 $\mu$M, more preferably lower than 33 $\mu$M, most preferred lower than 25 $\mu$M. A suitable method for determining the $IC_{50,HAI}$ is described in example 5 below.

Surprisingly, the inventors also achieved a broadening of the binding specificity of the peptide to hemagglutinin variants of different influenza strains and serotypes. In this way, the peptide of the present inventions offers better control of influenza infections in therapeutic and/or diagnostic applications due to a lowered risk of escaping viruses by antigenic drifting. Accordingly, the above-mentioned peptide affinity to influenza hemagglutinin and virus inhibition capability can comprise at least one, preferably at least two, most preferably at least three hemagglutinin serotypes selected from a group comprising H1, H3, H5 and H7, such as A/Aichi/2/68 (H3N2), A/NewYork/55/2004 (H3N2), A/Panama/2007/1999 (H3N2), A/Victoria/210/2009 (H3N2), A/Puerto Rico/8/1934 (H1N1), A/Vietnam/1203/2004 (H5N1), A/Mute swan/Germany/R901/06 K3141 (H7N1).

The peptide can consist of said 8 to 40 amino acids, and furthermore $X^1$ and/or $X^6$ can be Phe.

In a variant of reference, the peptide comprises the sequence
$X^1$-$X^2$-$X^3$-Tyr-Asp-$X^5$-$X^6$-$X^7$ (SEQ ID NO:3).

In preferred variants of reference, the peptide comprises the sequence

$X^1$-$X^2$-$X^3$-Tyr-Asp-Val-$X^6$-$X^7$ (SEQ ID NO: 4) or
$X^1$-Tyr-$X^3$-Tyr-Asp-$X^5$-$X^6$-$X^7$ (SEQ ID NO:5).

In further preferred variants of reference, the peptide comprises the sequence

$X^1$-Tyr-$X^3$-Tyr-Asp-Val-$X^6$-$X^7$ (SEQ ID NO:6),
$X^1$-Tyr-$X^3$-Tyr-Asp-$X^5$-Phe-$X^7$ (SEQ ID NO:7) or
$X^1$-$X^2$-$X^3$-Tyr-Asp-Val-Phe-$X^7$ (SEQ ID NO:8).

The sequence is selected from a group consisting of:

Phe-Tyr-$X^3$-Tyr-Asp-Val-$X^6$-$X^7$ (SEQ ID NO:9)
$X^1$-Tyr-$X^3$-Tyr-Asp-Val-Phe-$X^7$ (SEQ ID NO:10),
Phe-$X^2$-$X^3$-Tyr-Asp-Val-Phe-$X^7$ (SEQ ID NO:11), and
Phe-Tyr-$X^3$-Tyr-Asp-$X^5$-Phe-$X^7$ (SEQ ID NO:12).

In certain variants, the sequence is further selected from a group consisting of:

Phe-Tyr-$X^3$-Tyr-Asp-Val-Phe-$X^7$ (SEQ ID NO:13),
Phe-Tyr-$X^3$-Tyr-Asp-Val-$X^6$-Tyr (SEQ ID NO:14), and
Phe-Tyr-$X^3$-Tyr-Asp-$X^5$-Phe-Tyr (SEQ ID NO:15).

With the above variants, the inventors accomplished a much stronger binding and a broadened specificity of the peptide to hemagglutinin H1, H3, H5 and H7 of various influenza A serotypes such as H1N1, H3N2, H5N1 and H7N1 than the naturally evolved sequence SEQ ID NO:1. In addition, these variants are efficiently capable of inhibiting infection with influenza viruses in vitro, whereas the original sequence SEQ ID NO:1 does not exhibit an inhibitory effect.

In some variants, the sequence is selected from a group consisting of:

$X^1$-Tyr-Asp-Tyr-Asp-Val-Phe-Tyr (SEQ ID NO:16),

Phe-X²-Asp-Tyr-Asp-Val-Phe-Tyr (SEQ ID NO:17),
Phe-Tyr-X³-Tyr-Asp-Val-Phe-Tyr (SEQ ID NO:18),
Phe-Tyr-Asp-X⁴-Asp-Val-Phe-Tyr (SEQ ID NO:19),
Phe-Tyr-Asp-Tyr-Asp-X⁵-Phe-Tyr (SEQ ID NO:20),
Phe-Tyr-Asp-Tyr-Asp-Val-X⁶-Tyr (SEQ ID NO:21), and
Phe-Tyr-Asp-Tyr-Asp-Val-Phe-X⁷ (SEQ ID NO:22).

The present inventors surprisingly and unexpectedly found that these singly substituted variants do not only allow achieving a higher affinity to influenza hemagglutinin, but also allow tailoring the specificity of the peptide binding to different influenza A virus serotypes and subtypes. In this way, the binding strength of the peptide can, for example, be used as a characteristic marker for distinguishing between different influenza serotypes.

In any of the above-mentioned variants, at least one of
$X^1$ is selected from the group consisting of Leu, His, Ile, Trp, Asp, Glu, Gly, Lys, Asn, Pro and Arg;
$X^2$ is selected from the group consisting of Leu, Pro, Gln, Arg, Cys, Asp, Glu, Ile, Lys, Met and Ser;
$X^3$ is selected from the group consisting of Tyr, Gly, Phe, Gly, Gln, Thr, Trp and Leu;
$X^4$ is selected from the group consisting of Ile, Ala, Cys, Asp, Glu, Leu, Met, Pro, Val and Thr;
$X^5$ is selected from the group consisting of Pro, Gln, Asp, Glu, Arg;
$X^6$ is selected from the group consisting of Ala, Asp, Pro, Cys, Glu, Lys, Gln, Gly and Thr; and
$X^7$ is selected from the group consisting of Phe, Asn, Cys, Asp, Glu, Met, Pro, Leu, Thr, Trp and Ser.

In further preferred variants, at least one of
$X^1$ is Phe or Leu, preferably Phe;
$X^2$ is Tyr or Leu, preferably Tyr;
$X^3$ is Asp, Tyr or Gly, preferably Gly;
$X^4$ is Tyr or Pro, preferably Tyr;
$X^5$ is Val, Pro, Arg or Gln, preferably Val;
$X^6$ is Lys, Gln, Phe or Ala, preferably Lys or Phe; and
$X^7$ is Tyr or Phe, preferably Phe.

In some preferred variants, the peptide comprises the sequence selected from a group consisting of SEQ ID NO:23 to SEQ ID NO:98.

In preferred variants, the peptide further comprises N-terminally adjacent to any of the above-mentioned sequences a sequence Ala-Arg-Asp and/or C-terminally adjacent to any of the above-mentioned sequences the sequence Tyr-Ala-Met-Asp. In this way, the inventors accomplished enhanced solubility and bioavailability of the peptide.

In some variants, the peptide further comprises an (oligo-)lysine $(Lys)_n$ with $1 \leq n \leq 8$, preferably $2 \leq n \leq 6$. The (oligo-)lysine imparts improved solubility to the peptide and, at the same time, provides a convenient handle for enhanced coupling of the peptide to a functional group by electrostatic interaction or by forming a covalent bond, such as an amide bond. The coupling can for example include immobilization of the peptide on a solid support or surface, for instance via an, optionally activated, carboxylic acid or an epoxide. Preferably, the (oligo-)lysine forms the C- or N-terminus of the peptide, however, it is also possible that the (oligo-)lysine is nonterminally present, i.e. within the peptide sequence.

The peptide can include molecules which are not naturally occuring in proteins, such as non-natural amino acids, spacer molecules and/or linker molecules. A spacer molecule shall mean any non-branched molecule included in the peptide and providing a connection between two other parts of the peptide or between the peptide and a support, wherein the connection has a linear length of at least 15 Å, preferably at least 20 Å, most preferably at least 25 Å. One example is a polyethylene glycol molecule of at least two ethylene oxide units, preferably at least four ethylene oxide units, most preferably at least ten ethylene oxide units. When used as a spacer for tethering the peptide to a support, polyethylene glycol adds flexibility to the tether and improves viral attachment to the supported peptide by remedying steric constrains. In medical applications, including polyethylene glycol into the peptide can improve the solubility and reduce the immunogenicity of the peptide. Moreover, polyethylene glycol can improve the pharmacokinetic properties of the peptide by reducing renal clearance and resulting in more sustained absorption after administration. A linker molecule shall mean any molecule included in the peptide and capable of forming a chemical and/or physical connection to another molecule or entity. Typical examples are biotin, hydrazine, and sulfhydryl linkers as well as polymerizable linkers such as vinylidene derivatives. Other suitable spacer and linker molecules can be determined without difficulty (see, for example G. T. Hermanson "Bioconjugate Techniques", J. Audet and M. Preap (eds.), Elsevier Academic Press 2013, UK).

It is preferred that the peptide has less than 30, preferably less than 25, more preferably less than 20, most preferred less than 15 amino acids. Such peptides are about 50 to 100 times smaller in terms of molecular weight than an antibody. Whilst development, production and quality of antibodies is expensive and time-consuming, the short peptides of the present invention can, for instance, be fully synthetically produced in large scales with additional ease of purification and quality control at considerably lower cost. Without limitation, a preferred method for producing the peptide is established solid-phase peptide synthesis using the Fmoc- or Boc-strategy.

In a variant, at least a part of the peptide is cyclized by forming a ring generated by a covalent bond linking an N-terminal and a C-terminal moiety, an N-terminal moiety and a side chain moiety, a C-terminal moiety and a side chain moiety, or two side chain moieties of the peptide. As used herein, the term moiety shall mean an amino acid in general and can specifically include an alpha-amino group on the N-terminus of the peptide and an alpha-carboxy group on the C-terminus of the peptide as well as amino, carboxy and thiol groups in an amino acid side chain. Moreover, the term moiety shall be understood as including any functional group which can be incorporated into the peptide to form an intramolecular bridge with a complementary functional group, such as a thiol, azide or alkyne group. Preferably, the cyclized part includes at least part of, more preferably the complete sequence described above. Suitable methods for generating cyclic peptides are, for instance, described in "Fmoc Solid phase peptide synthesis: A practical approach", W.C. Chan and P.D. White (eds.), Oxford University Press 2000. Without being bound by theory, the cyclized peptide exhibits a higher affinity to hemagglutinin, which is attributed to a higher rigidity compared to the corresponding linear form. Another advantage is that the cyclized form imparts an improved resistance to enzymatic digestion, which enables the peptide to survive in the human digestive tract after oral administration and also improves its pharmacokinetic properties.

In a further variant, the peptide comprises at least one D-amino acid. The incorporation of a D-amino acid makes the peptide highly resistant to degradation by proteases. In specific variants, at least 50%, at least 70%, at least 90% or all of the amino acids are D-amino acids.

In a second aspect, this invention provides a peptide as described above or a pharmaceutically acceptable salt thereof for use as a medicament. Preferably, the peptide is used in the prevention and/or treatment of influenza A virus infection in a subject.

Without being bound by theory, the peptide of the present invention binds within the receptor binding site of hemagglutinin to a region which is conserved among several HA serotypes. This does not only inhibit binding of the virus to its cellular receptor but also inhibits fusion of the virus with the cellular membrane and infecting living cells. In this way, the inventors accomplished a broad anti-viral activity against a variety of influenza strains. Remarkably, this anti-viral activity has proven to be effective despite several decades of antigenic drifting of a virus strain.

As used herein, treatment shall mean slowing, stopping or reversing the progression of the influenza virus infection. Prevention, as opposed to treatment, shall mean avoiding the outbreak of influenza virus infection or limiting the severity of the infection in a subject which has been exposed to the virus.

As used herein, subject shall mean any animal, for example human, non-human primate, swine and avian subjects. Preferably, the subject is human.

The peptide can be comprised in a pharmaceutical composition or used for the manufacture of a pharmaceutical composition. The pharmaceutical composition preferably comprises a therapeutically effective amount of the peptide. The pharmaceutical composition can contain further additives, such as water, salts, e.g. sodium chloride, lactose, cellulose, starch, saccharose, vegetable oil, paraffin, hard fat, polyethylene glycol and/or polyethylene oxide as well as derivatives thereof. The pharmaceutical composition can also comprise a protease inhibitor. The pharmaceutical composition can be adapted for local, systemic or external application.

The influenza A virus is preferably selected from a group comprising the H1, H3, H5 and H7 serotype, such as H3N2, H1N1, H5N1 and H7N1.

In a third aspect, the invention provides a composition comprising a plurality of peptides according to the first aspect and a supporting material to which the plurality of peptides is physically and/or chemically bonded.

The inventors realized that the relatively dense packing of hemagglutinin molecules on the influenza virus surface offers means for multivalent binding of the virus. By arranging a plurality of peptides on a supporting material, the inventors achieved simultaneous binding between several peptides and hemagglutinin molecules on the viral surface. In this way, a cooperative binding effect is obtained, which results in a high avidity binding of the influenza virus with the composition. Furthermore, due to the multivalent peptide display on the supporting material and the associated high effective peptide density, the composition also has both kinetically and thermodynamically improved binding properties in comparison to a naturally derived antibody.

The supporting material can be of a variety of types. For example, the supporting material can comprise an organic or inorganic nano- or microparticle. The supporting material can comprise a lipid layer, preferably a lipid bilayer, for instance a liposome or micelle. The supporting material can comprise a natural and/or synthetic polymer. Suitable polymers include polyglycerol, polysaccharide, e.g. dextran, polyurethane, polyurea, polyester, polyamide, polystyrol, polyvinyl, aminoplast, epoxides, silicones, polyethylenglycol, acrylates, cellulose derivatives, parylenes, copolymers or mixtures thereof. The supporting material can also include a surface, for example a functionalized or non-functionalized glass or plastic surface or a metallic surface, such as a gold surface.

In a preferred embodiment, the composition comprises a molecularly imprinted polymer obtainable by a polymerization reaction including a plurality of peptides having a polymerizable group in the presence of a plurality of at least parts of influenza A hemagglutinin, more preferably in the presence of at least a part of an influenza A virus particle comprising a plurality of hemagglutinin molecules such as an, preferably inactivated, influenza A virus.

The polymerizable group can be any moiety which allows reacting the peptides in a polymerization reaction. Typically, the polymerizable group is a reactive moiety which undergoes a cross-linking reaction, for example with an inorganic and/or organic cross-linker. This cross-linking reaction is preferably a polymerization reaction, the term "polymerization" herein being understood as also including a polycondensation or polyaddition. The polymerizable group can, for instance, be chosen from the group comprising vinyl, acrylic, methacrylic, allyl or styrene or any other unsaturated group capable of reacting via a free-radical process, and chemical groups enabling a polycondensation, polyaddition or sol-gel reaction. Examples of suitable polymerizable groups include acryl derivatives and methacryl derivatives such as acrylic acid, methacrylic acid, ethylene glycol dimethacrylate or trimethylolpropane trimethacrylate, epoxides, isocyanates or allyl derivatives. Such polymerizable groups can for example be conveniently incorporated as part of the peptide during solid phase synthesis.

Accordingly, a method for preparing the molecularly imprinted polymer includes the following method steps:

I) providing a plurality of peptides according to the first aspect having a polymerizable group and a plurality of at least parts of influenza A hemagglutinin such as at least part of an influenza A virus particle comprising a plurality of hemagglutinin molecules,

II) forming a pre-polymerization mixture by bringing said plurality of peptides and said plurality of at least parts of influenza A hemagglutinin into contact,

III) polymerizing said pre-polymerization mixture to obtain the molecularly imprinted polymer.

Preferably, at least one cross-linker and/or initiator is added to the pre-polymerization mixture of method step II). Any type of cross-linker known in the state of the art in this matter may be used, for example acryl derivatives, methacryl derivatives, e.g. ethylene glycol dimethacrylate or trimethylolpropane trimethacrylate, or allyl derivatives, bifunctional or multifunctional organosiloxane, or combinations thereof. To achieve a higher hydrophilicity of the polymer backbone, corresponding cross-linkers based on polyethylene glycol can be used.

The method preferably comprises a further step IV) removing the target molecule from the polymer.

These compositions can be used with advantage in diagnostic applications, wherein the high avidity of the composition results in a favourably low detection limit of influenza virus. Such compositions can also be used in therapeutic applications in which the high avidity results in an almost irreversible binding of circulating viruses, thereby effectively inhibiting the viruses from binding, fusion and infection of a target cell.

In a fourth aspect, the present invention provides a method for diagnosing influenza A virus infection in a subject. The method comprises the following steps:

A) obtaining a sample of body material from the subject,

B) distinguishably contacting the sample with i) at least one of the peptides according to the first aspect and ii) a control lacking the at least one peptide,

C) detecting binding to the at least one peptide and to the control,

wherein an elevated binding to the at least one peptide in comparison to the control is indicative of influenza A virus infection in the subject.

As used herein, body material shall be understood as including body fluid such as any fluid that is excreted or secreted from the body. Typically, the body fluid is blood, serum, saliva, tear fluid, lymphatic fluid, urine or sweat. Body material shall also include tissue samples, for instance mucosa samples, such as samples from oral mucosa.

Preferably, the method is carried out by providing the at least one peptide and the control distinguishably immobilized on a solid support, such as in separate wells of a microplate, or in pre-determined positions of a membrane, glass or polymer support, for example in different spots of a microarray.

The detection of binding is not particularly limited and can be accomplished with any method known in the art for this matter. For instance, it is possible to use a suitably labelled monoclonal or polyclonal antibody to detect influenza A virus bound to the peptides, or a sandwich system with an unlabelled primary antibody which binds to the virus and a labelled secondary antibody which binds to the primary antibody. The label can be any label detectable with methods known in the art for this purpose, for example, a fluorescence label, an electrochemical label, or an enzyme label which catalyzes a colorimetric, chemiluminescent, gravimetric or electrochemical reaction. The label can also include a nanoparticle, such as a gold or silver nanoparticle or a quantum dot. The binding can also be detected by so-called label-free methods such as surface plasmon resonance, quartz crystal microbalance, total internal reflection, microscale thermophoresis, surface acoustic waves or calorimetry.

The control can be any suitable control which itself does not specifically bind to influenza A virus and therefore allows determination of specific binding of influenza A virus to the at least one peptide. Preferably, the control is similar in physico-chemical properties to the at least one peptide, e.g. in terms of molecular weight, net charge, hydrophilicity and/or hydrophobicity. Preferred controls include sequence variations of the at least one peptide, such as inverted and/or permutated sequences. Other suitable controls can be determined by the skilled person without any difficulty.

It is also possible to contact the sample in method step Bi) with a mixture of two or more peptides according to the first aspect which differ in sequence from each other in at least one amino acid. In this way, the preferential binding of the peptides to different virus subtypes can be advantageously combined in order to achieve broad specificity across various influenza A strains and, thus, high sensitivity of the method in diagnosing influenza A infection in general.

In a fifth aspect, the present invention provides a method for determining the type of an influenza A virus. The inventors surprisingly discovered that different variants of the peptide of the present invention bind different influenza A virus serotypes with a characteristic strength. In this way, the virus binding of peptides differing in at least one amino acid can be used as a reliable indicator of the virus serotype by relating the virus binding of at least two of the above-defined peptides to each other. The characteristic relationship derived therefrom can then be compared with reference relationships derived from binding of predetermined influenza A viruses to the same peptides. Typically, the type of the influenza A virus corresponds to that of the most closely matching reference relationship. Accordingly, the method comprises the following steps:

a) providing at least a first and a second peptide according to the first aspect, wherein the first and second peptide have a predetermined sequence differing in at least one amino acid from each other,

b) distinguishably contacting the first and second peptide with a sample containing influenza A virus material,

c) detecting binding of the influenza A virus material to the first and second peptide,

d) correlating the binding to the first peptide with the binding to the second peptide,

e) matching the correlation with a reference correlation of the binding to the first and second peptide of material of a known type of influenza A virus.

The method can also comprise contacting at least three, at least 5 or at least 10 of the above-described peptides having a predetermined sequence and differing in at least one amino acid from each other. In this way, the individual capacity of each of the peptides to bind the influenza virus material results in a highly characteristic binding correlation, from which the influenza A virus type can be derived with high accuracy similar to a fingerprint.

Preferred embodiments for carrying out the method of the fifth aspect correspond to those of the fourth aspect. In a particularly preferred embodiment, the peptides are individually arranged in pre-determined positions in a microarray format. In this way, the method can be advantageously performed within a single analysis requiring only a minimum amount of virus material.

Brief description of the figures

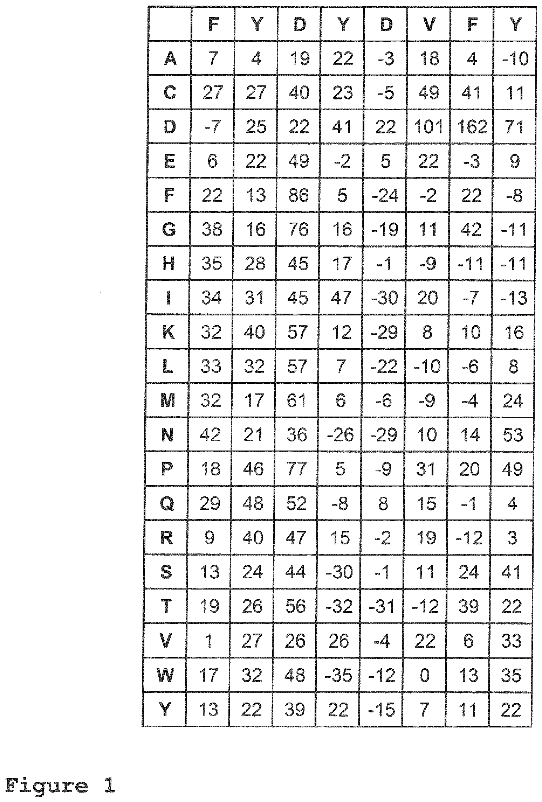

Figure 1    shows the relative influenza A virus X31 (A/Aichi//2/68 H3N2) binding strength in % of different peptide variants in comparison to the binding of the virus to fetuin serving as a surrogate of the virus' host cell receptor, set to 0%. The peptide variants each bear a single amino acid substitution in comparison to SEQ ID NO:1, wherein each amino acid of SEQ ID NO:1 (indicated by the top row) was substituted with each of the remaining natural amino acids (indicated by the left column). A positive value indicates stronger virus binding, a negative value weaker virus binding in comparison to fetuin.

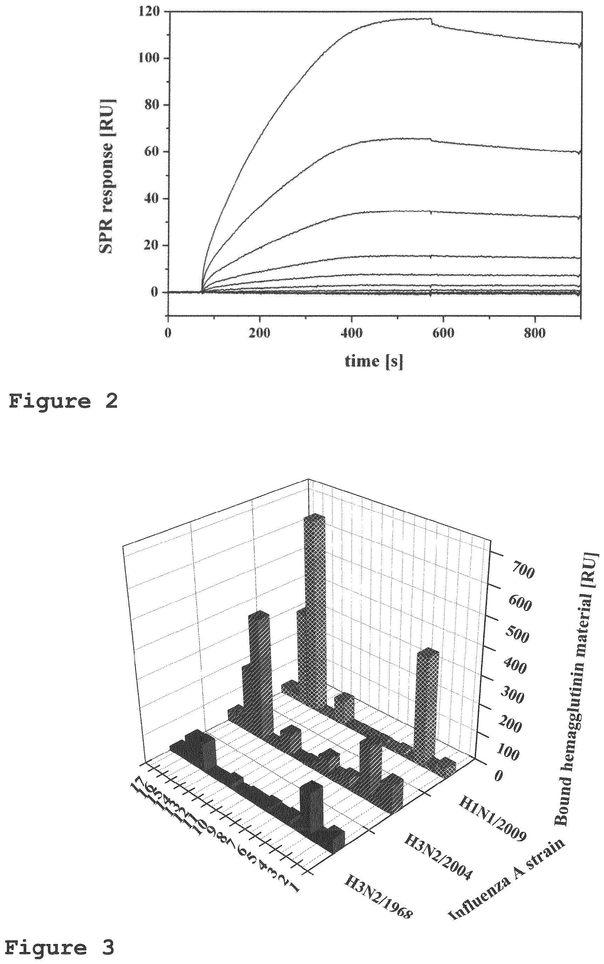

Figure 2    shows response curves of real-time surface plasmon resonance binding experiments, wherein X31 virus (A/Aichi//2/68 H3N2) was injected at different concentrations over a surface modified with peptide SEQ ID NO:1.

Figure 3    is a column diagram showing the maximum binding of influenza strains H3N2/1968, H3N2/2004 and H1N1/2009 on peptide modified surfaces in surface plasmon resonance binding experiments. 1: SEQ ID NO:1, 2: SEQ ID NO:44, 3: SEQ ID NO:23, 4: SEQ ID NO:24, 5: SEQ ID NO:102, 6: SEQ ID NO:25, 7:

SEQ ID NO:26, 8: SEQ ID NO:76, 9: SEQ ID NO:79, 10: SEQ ID NO:92, 11: SEQ ID NO:27, 12: SEQ ID NO:94, 13: SEQ ID NO:87, 14: SEQ ID NO:28, 15: SEQ ID NO:29, 16: SEQ ID NO:30, 17: SEQ ID NO:31.

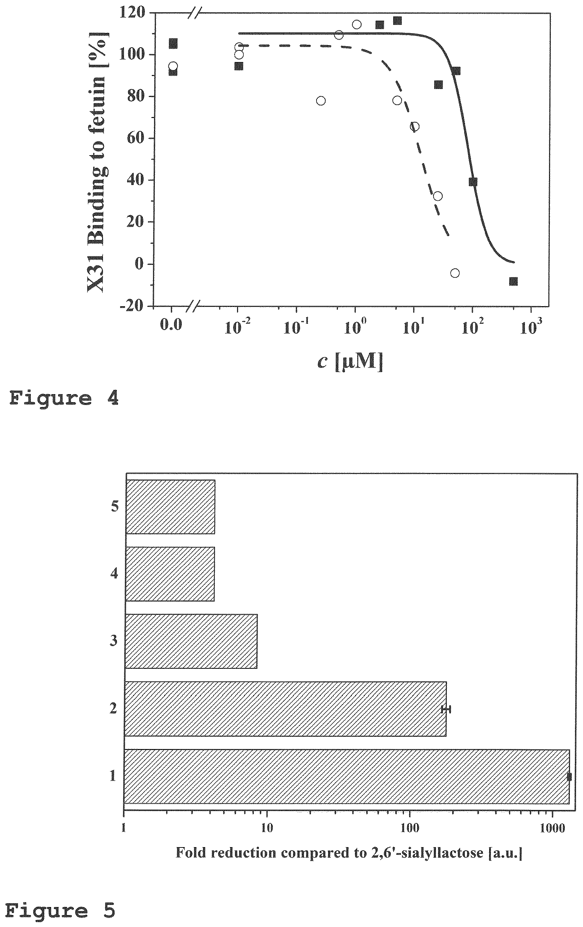

Figure 4     shows dose-response curves of the ability of the peptide of the present invention with sequence SEQ ID NO:28 (dashed line, circle markers) in comparison to the reference peptide with sequence SEQ ID NO:1 (solid line, square markers) to inhibit the influenza A virus X31 from binding to immobilized fetuin, as determined by surface plasmon resonance.

Figure 5     is a bar diagram showing the reduction of the $K_i$(HAI) value relative to 2,6'-sialyllactose of the peptide of the present invention with SEQ ID NO:28 (1) and the reference peptide with SEQ ID NO:1 (2) in comparison to hemagglutinin binding peptides reported in the prior art with SEQ ID NO:100 (3), SEQ ID NO:101 (4) and SEQ ID NO:102 (5).

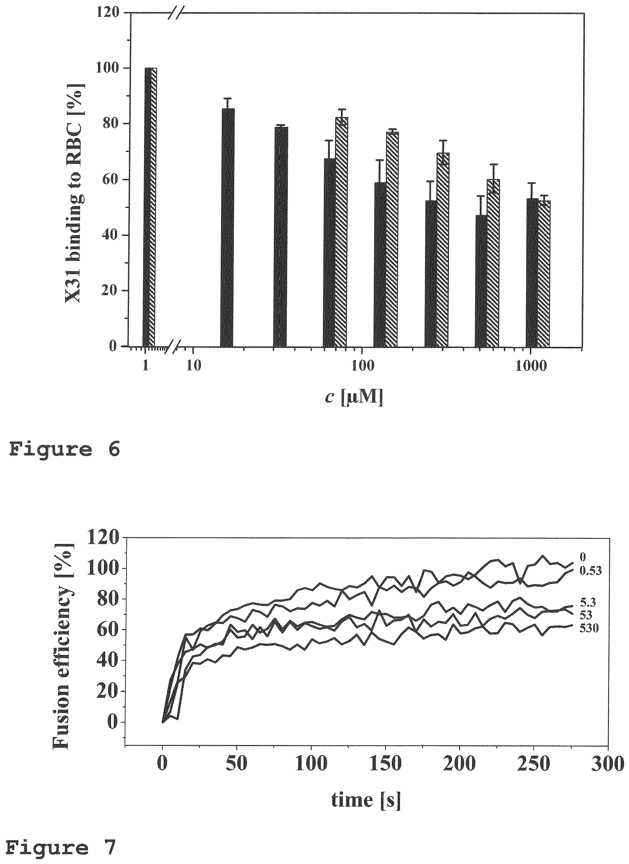

Figure 6     is a column diagram showing the binding inhibition of intact X31 viruses to single human red blood cells by the peptide according to the present invention with SEQ ID NO:28 (solid columns) in comparison to the reference peptide with SEQ ID NO:1 (hatched columns), as determined by flow cytometry experiments.

Figure 7     is a line plot of real-time measurements of the fusion efficiency in % over time of virus X31 with erythrocyte ghosts in the presence of different concentrations of the peptide of the present invention with SEQ ID NO:28.

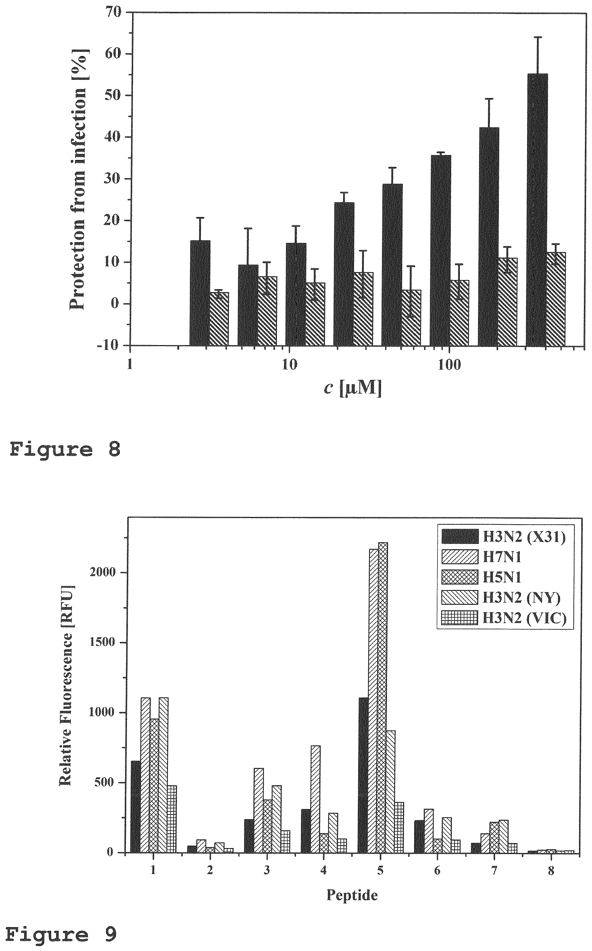

Figure 8     is a column diagram showing the dose-dependent protection of host cells from virus infection in % with the peptide of the present invention with SEQ ID NO:28 (solid columns) and the reference peptide with SEQ ID NO:1 (hatched columns).

Figure 9     is a column diagram showing the fluorescence intensities measured in a microarray experiment after binding of various fluorescence-labelled influenza A virus strains and subtypes to different peptides of the present invention distinguishably immobilized in discretely ordered, micro-sized test features (spots) on a glass surface; 1: SEQ ID NO:28, 2: SEQ ID NO:24, 3: SEQ ID NO:32, 4: SEQ ID NO:33, 5: SEQ ID NO:97, 6: SEQ ID NO:98, 7: SEQ ID NO:34, 8: buffer control.

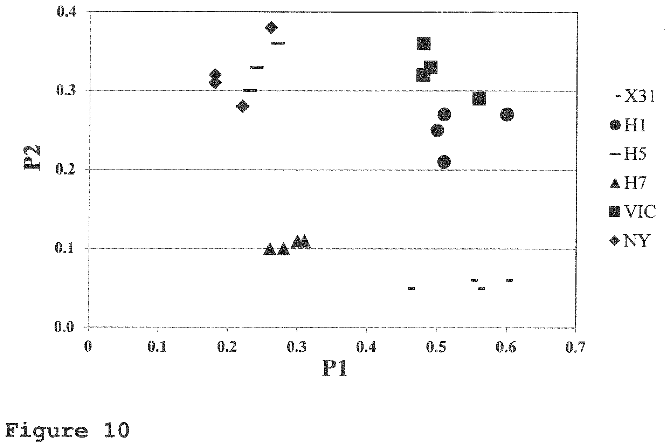

Figure 10     is a two-dimensional symbol plot of relative binding of various influenza A virus strains to the immobilized peptide with SEQ ID NO:33 (P1, x-axis) in relation to the immobilized peptide with SEQ ID NO:28 (P2, y-axis) determined by microarray experiments with fluorescence-labelled viruses. The binding values represent the scaled fluorescence intensity vectors.

Brief description of the sequences

SEQ ID NO:1 is a reference peptide sequence.

SEQ ID NO:2 to SEQ ID NO:98 are peptide sequences according to the present invention.

SEQ ID NO:99 to SEQ ID NO:102 are comparative peptide sequences according to the prior art.

SEQ ID NO:103 and SEQ ID NO:104 are reference peptide sequences.

Detailed description of embodiments

In the following, certain embodiments of the invention are described in more detail with reference to figures and experimental data. The examples and figures are not intended to be limiting with respect to specific details.

Example 1

Prediction of linear hemagglutinin binding peptides

The structure of the Fab-hemagglutinin complex with PDB accession number 2vir (Fleury et al., Nat. Struct. Biol., 1989, 5, 119-123) was used as a basis for predicting linear peptide sequences within the antibody molecule which contribute to hemagglutinin binding.

The analysis resulted in the identification of a linear peptide (ARDFYDYDVFYYAMD; SEQ ID NO:103) which appeared to associate strongly with hemagglutinin. The central binding motif was determined between residues 4-11, i.e. the sequence FYDYDVFY (SEQ ID NO:1).

Example 2

Systematic sequence improvement by substitutional analysis

In order to experimentally verify the results of the prediction, a full substitutional analysis was performed in which each of the eight amino acids of SEQ ID NO:1 was substituted with each of the 19 remaining canonical amino acids.

The experiments were performed in a microarray format. A PepStar peptide microarray spotted on glass slides covering the one hundred fifty-nine resulting peptide sequences was purchased from JPT (Berlin, Germany). X31 virus was inactivated by UV-irradiation and labelled with fluorescent dye 634 ($\lambda_{ex}$ = 635 nm, $\lambda_{em}$ = 654 nm) using the Fluoro-spin 634 kit (emp Biotech, Berlin, Germany) according to the manufacturer's instructions. The labelled X31 virus was diluted in Femtotip buffer (20 mM Tris, 30% glycerol, 3% polyvinylpyrrolidon 90, 0.1% Tween 20, pH 8.4) and incubated on the microarray overnight. Afterwards, the microarrays were washed twice with Femtotip buffer and twice with ultrapure water and subsequently dried under a stream of nitrogen. Fluorescence was detected using an Axon 4200 A laser scanner (Molecular Devices, Biberach, Germany). Fluorescence intensity was extracted using GenePix Pro 6.0 software. The extent of binding of the virus to each individual peptide was calculated as the contrast value C according to the equation

$$C = \frac{I_S - I_{NC}}{I_S + I_{NC}},$$

wherein $I_S$ represents the relative fluorescence intensity measured in a spot containing a specific peptide and $I_{NC}$ represents the relative fluorescence intensity of a spot containing a negative control. The contrast values were further expressed in terms of a percentage relative to the positive control fetuin, which was set to 0%.

The results are shown in Figure 1. The experiments revealed that the aspartic acid in position 5 of the sequences is essential for the binding of the peptides to the virus. Conversely, for example, the substitution of the aspartic residue in position 3 of the peptide almost generally leads to an improved binding, whereas substitution of the tyrosine in position 4, valine in position 6 and phenylalanine in position 7 except for some substitutions reduces the binding capacity. The peptides which exhibited a favourable binding ability are listed as SEQ ID NO:35 through SEQ ID NO:96.

Example 3

Affinity and specificity engineering by combinatorial substitutions

In the next phase, selected amino acid substitutions were combined in order to achieve a further improvement of affinity and specificity of the peptide across different influenza A virus strains. For this purpose, peptides with the SEQ ID NO:1, SEQ ID NO:23, SEQ ID NO:24, SEQ ID NO:25, SEQ ID NO:26, SEQ ID NO:27, SEQ ID NO:28, SEQ ID NO:29, SEQ ID NO:30, SEQ ID NO:31, SEQ ID NO:44, SEQ ID NO:76, SEQ ID NO:79, SEQ ID NO:87, SEQ ID NO:92, SEQ ID NO:94 and SEQ ID NO:102 were synthetically produced by standard Fmoc solid phase synthesis as C-terminal peptide amides and N-terminally equipped with an oligo-lysine (Lys)$_4$. The quality of the peptides was controlled and verified by HPCL and mass spectrometry and the peptides were used in a purity of at least 95%.

The measurements were performed on a surface plasmon resonance biosensor (SPR) using a Biacore T100 facility with T200 sensitivity enhancement (GE-Healthcare Bio-Sciences AB, Uppsala, Sweden). The system was operating at 25 °C with HBSP running buffer (GE-Healthcare) at a flow rate of 1-10 μl/min for peptide immobilization or 10-30 μl/min for binding studies. The peptides were immobilized on CM5 sensor chips (GE-Healthcare) using amine coupling with EDC/NHS at a flow rate of 10 μl/min according to the manufacturer's instructions. For SPR measurements, influenza strains A/Aichi/2/68 H3N2 (X31), A/California/7/2009 H1N1 and A/NewYork/55/2004 H3N2 were used as analytes after inactivation by UV-irradiation for 5 min on ice. Kinetic binding experiments were performed by injecting several virus concentrations between 0 and 50 μg/ml over the peptide functionalized surfaces allowing 500 seconds association time and 600 seconds disassociation time, followed by a 60 seconds regeneration step with 50 mM NaOH at a flow rate of 10 μl/min. Quantitative binding of virus material was determined 10 seconds after stopping virus injection as the mean binding response over the next 5 seconds dissociation time.

Figure 2 shows a representative surface plasmon resonance sensorgram for the binding of X31 virus to a surface displaying the peptide with SEQ ID NO:1. Steady state affinity calculation indicated an equilibrium dissociation constant $K_D$ of 59.0 $\mu$M.

An overview of the SPR analysis of the binding ability of the investigated peptide sequences to all three tested influenza A virus strains is shown in Figure 3. The x-axis displays the peptide sequence, the y-axis indicates the respective influenza strain and the z-axis shows the SPR response at maximum binding. The inventors surprisingly found that the amino acid substitutions in the different peptide variants correlate with a binding preference to a specific virus type. For example, whilst the native peptide with SEQ ID NO:1 (col. 1) has a preference for influenza A virus strain H3N2/2004, the substitution of the Asp residue in position 3 (for instance col. 3: SEQ ID NO:23, col. 14: SEQ ID NO:28, col. 15: SEQ ID NO:29) shifts the binding preference strongly to H1N1/2009. Conversely, the substitution of the Val residue in position 6 by arginine (col. 8: SEQ ID NO:76) creates a peptide which specifically binds the virus H3N2/2004 but does not bind the other two virus types.

Some peptides, particularly those involving combined substitutions, for instance of Asp in position 3 and Phe in position 8, result in a significantly increased binding strength to all three influenza viruses. As one representative example, the peptide with SEQ ID NO:28 (col. 14), wherein the Asp in position 3 is replaced by Gly and the Tyr in position 8 is replaced by Phe, has a higher affinity to H3N2/1968 (X31) in comparison to the native peptide with SEQ ID NO:1 (col. 1), which is evident from the by a factor of 2 increased binding signal. Remarkably, the binding capability of this variant is even more enhanced for H3N2/2004 (factor of 4) and much further enhanced for H1N1/2009 (factor of 24).

Example 4

Inhibition of influenza A virus binding to fetuin

Since the peptides of the present invention were designed to bind to the receptor binding site of influenza A virus hemagglutinin, it was investigated whether the peptides are able to inhibit binding of an influenza A virus to fetuin. Fetuin is an accepted surrogate of the virus' natural cell surface receptor which is an N-acetyl neuraminic acid residue terminally linked by an $\alpha$-2,3 or $\alpha$-2,6 bond to a carbohydrate moiety of a glycoprotein or glycolipid.

To this end, surface plasmon resonance measurements were performed as described in example 3, wherein the chip surface was modified with fetuin (Sigma-Aldrich, Taufkirchen, Germany). Constant concentrations of 50 or 100 $\mu$g/ml X31 virus (A/Aichi/2/68 H3N2) were used together with a varying concentration of the peptides. The solution of virus and peptide was mixed and subsequently incubated for at least 15 min to reach equilibrium before the mixture was injected over the SPR surface.

As a representative result, Figure 4 shows the dose dependent binding inhibition of X31 to fetuin for the peptide with SEQ ID NO:28 of the present invention in comparison to the reference peptide with SEQ ID NO:1. Whilst both peptides have the ability to inhibit the binding of the X31 virus to its receptor, the peptide of the present invention has a significantly improved inhibition potency in comparison to the natural counterpart, exemplified by an about 4.5-fold lower $IC_{50,RB}$ of 20 $\mu$M in comparison to 90 $\mu$M of the reference peptide.

Example 5

Hemagglutination Inhibition Assay

The hemagglutination inhibition assay (HAI) relies on the fact that hemagglutinin agglutinates red blood cells but is inhibited in a dose-dependent manner when a peptide is present that can interfere with the virus attachment to the red blood cells.

For this experiment, the peptides were two-fold serially diluted in phosphate buffered saline. Afterwards, two hemagglutination units (HAU) containing $2 \cdot 10^7$ virus particles (A/Aichi/2/68 H3N2, A/Panama/2007/1999 H3N2 or A/Mute swan/Germany/R901/06 K3141 H7N1) were added to each dilution and 30 minutes incubation at room temperature was maintained. Next, 50 $\mu$l of an erythrocyte solution with approximately $2 \cdot 10^6$ cells/$\mu$l was added, gently mixed and incubated for 60 minutes at room temperature. The H3N2 hemagglutination inhibition assay included human erythrocytes, whereas the H7N1 hemagglutination inhibition assay included turkey erythrocytes. The inhibitor constant $K_i$(HAI) reflects the lowest inhibitor concentration which is necessary to achieve 100% inhibition. $IC_{50,HAI}$ values represent the half maximal inhibitory concentration between the $K_i$(HAI) and full hemagglutination.

As a representative example, the $K_i$(HAI) results of the peptide with SEQ ID NO:28 of the present invention and the reference peptide with SEQ ID NO:1 are summarized in Table 2.

Table 2: Mean $K_i$(HAI) values in $\mu$M of the reference peptide with SEQ ID NO:1 and the peptide with SEQ ID NO:28 of the present invention for different influenza A viruses. Values in parentheses are standard deviations of triplicates.

| | A/Aichi/2/68 H3N2 | A/Panama/2007/199 9 H3N2 | A/MuteSwan/Germany/R901/06 H7N1 |
|---|---|---|---|
| SEQ ID NO:1 | 235 (15) | 16 (-) | 62.5 (10.4) |
| SEQ ID NO:28 | 31.9 (0.7) | 0.1 (-) | 36.5 (9.8) |

The peptide with SEQ ID NO:28 exhibited for all tested virus strains a significantly stronger inhibitory effect than that of the reference peptide with SEQ ID NO:1.

For comparison, the $K_i$(HAI) values of hemagglutinin binding peptides reported in the prior art s2 (SEQ ID NO:100; Matsubara et al., J. Med. Chem. 2010, 53, 4441-4449), P1 (SEQ ID NO:101; Wu et al., PloS One, 2011, 6, e23058) and L-P1 (SEQ ID NO:102; Rajik et al. Int. J. Biol. Sci. 2009, 5, 543-548) were also determined (Figure 5). Notably, the $K_i$(HAI) of the peptide of the present invention with SEQ ID NO:28 is up to 1,600-fold lower than the Ki(HAI) of the conventional peptides and shows the superior potential of the peptides of the present invention for treating influenza A virus infection by inhibiting viral binding.

Table 3 further summarizes the inhibitory potency of the peptide with SEQ ID NO:28 in comparison to the reference peptide with SEQ ID NO:1 in terms of the $IC_{50,RB}$ value and $IC_{50,HAI}$ value. It can be seen that the peptide according to the present invention is a significantly better inhibitor than its natural counterpart.

Table 3: Inhibitory potency of the reference peptide with SEQ ID NO:1 and the peptide with SEQ ID NO:28 of the present invention in receptor binding $IC_{50,RB}$ (example 4) and hemagglutination $IC_{50,HAI}$ (example 5) in $\mu$M.

| Influenza A strain | Inhibition of binding to fetuin ($IC_{50,RB}$) | | Hemagglutination inhibition ($IC_{50,HAI}$) | |
|---|---|---|---|---|
| | SEQ ID NO:1 | SEQ ID NO:28 | SEQ ID NO:1 | SEQ ID NO:28 |
| A/Aichi/2/68 H3N2 | 90 | 20 | 163 | 18 |
| A/Mute swan/Germany/R901 /06 H7N1 | 40 | 30 | 47 | 23 |

Example 6

Single cell binding inhibition assay

The capacity of the peptides to prevent influenza virus binding to single cells, a fluorescence dequenching assay was performed as previously described (Papp et al., ChemBioChem 2011, 12, 887-895). Briefly, influenza A virus (A/Aichi/2/68 H3N2) was labelled with 20 $\mu$M octadecylrhodamine B chloride (R18) in phosphate buffered saline and free dye was removed by virus pelleting. Then, 5 $\mu$g labeled virus was incubated with 2-fold serial dilutions of peptides and an incubation for 30 minutes at room temperature was maintained. Next, the virus-peptide complex was mixed with 40 $\mu$l of a 1% erythrocyte solution and incubated for 30 min at room temperature. Unbound virus was removed by centrifugation for 10 min at 1500 g. The resuspended pellet was analyzed for R18 fluorescence by flow cytometry on a Beckton Dickinson FACScan flow cytometer. Data were acquired by using BD CellQuest. From each measurement, a single cell population was gated and median fluorescence was normalized to an untreated virus control.

Furthermore, 5 $\mu$g A/Aichi/2/68 H3N2 virus labelled with R18 (0.5 $\mu$g/$\mu$l virus) was incubated with peptides in 2-fold serial dilutions. After an incubation of 30 minutes at room temperature, human erythrocyte ghosts were added, followed by another 30 minutes incubation at room temperature. Afterwards, unbound viruses were removed by pelleting the ghosts at 300 g for 5 minutes. The resuspended pellet was transferred into glass cuvettes with acetate buffer (pH 7.5) at 37 °C and the fluorescence baseline was measured using a spectrofluorometer (Horiba Jobin Yvon FluoroMax). Fusion was triggered by decreasing the pH with citric acid to pH 5 and maximum fluorescence for normalization was determined by adding Triton X-100. Time-dependent fluorescence signals were averaged every 5 seconds.

The results are shown in Figures 6 and 7 representatively for peptide SEQ ID NO:28 in comparison to the reference peptide SEQ ID NO:1. It was found that the peptide of the present invention is able to inhibit the viral attachment in a dose-dependent manner up to 50% (Figure 6). Similarly, the kinetic measurement of viral fusion to the cell membrane of erythrocyte ghosts demonstrated that peptide SEQ ID NO:28 inhibits the fusion efficiency up to about 40% in a concentration-dependent manner (Figure 7).

These results strongly confirm a high potential of the peptides of the present invention in the treatment and prevention of influenza virus infections through their capacity to inhibit viral fusion to target cells.

Example 7

Infectivity inhibition assay

The effect of the peptides on viral cytopathicity was investigated. A viral cytotoxicity inhibition assay was performed using an MTS reagent (Promega). Here, $3 \cdot 10^4$ Madin-Darby Canine Kidney II (MDCK II) cells were seeded the day before infection. A/Aichi/2/68 H3N2 virus was pretreated with different peptides in a 2-fold dilution series for 30 min at room temperature under slight agitation. Cells were washed once with phosphate buffered saline including $Mg^{2+}$ and $Ca^{2+}$ and pretreated virus (MOI 0.05) was added for 1 hour at room temperature to allow binding. Unbound virus was removed by washing once with 37 °C warm infection medium (DMEM, 2 mM Glutamine, 0.1% FCS, 0.1% BSA, 2.5 $\mu$g TPCK, supplemented with penicillin and streptavidin), and subsequently incubated for 24 h at 37 °C. Two hours before the readout, 20 $\mu$l MTS solution was added to each dilution. After completion of the incubation, absorbance at 490 nm was measured and data were normalized to maximum viral toxicity.

Representative results are shown in Figure 8. The column diagram shows the protection from infection in percent in relation to the peptide concentration for peptide SEQ ID NO:28 of the present invention (solid columns) in comparison to reference peptide with SEQ ID NO:1 (shaded columns). No significant protection from viral infection was observed when the reference peptide was applied. Conversely, with the peptide of the present invention the inventors achieved a reduction of infectivity of up to about 55% in a concentration dependent manner. Therefore, the peptide of the present invention can be used for slowing, stopping or even reversing the progression of the influenza virus infection.

Example 8

Method for determining the influenza A virus type

Following the unexpected discovery of the inventors that different peptides of the present invention bind different influenza A virus serotypes and subtypes with a characteristic binding strength, it was investigated whether this unique property can be advantageously used in a method for determining the influenza A virus type.

The experiments were performed in a microarray format essentially as described in example 2 above. The peptide microarrays displayed, in individual spots, the peptide sequences of the present invention with SEQ ID NO:24, SEQ ID NO:28, SEQ ID NO:32, SEQ ID NO:33, SEQ ID NO:34, SEQ ID NO:97 and SEQ ID NO:98, each of which has either a single, double or triple amino acid substitution in comparison to SEQ ID NO:1. Fluorescently labelled virus strains H3N2 (A/Aichi/2/1968), H7N1 (A/Mute swan/Germany/R901/06 K3141), H1N1 (A/Puerto Rico/8/1934), H5N1 (A/Vietnam/1203/2004), H3N2 (A/Victoria/210/2009) and H3N2 (A/NewYork/55/2004) were used as analytes. The individual binding patterns obtained from binding of the specific fluorescently labelled viruses to the different peptides was used to apply mathematical models for the classification and prediction of influenza A virus strains.

Figure 9 shows the results of the binding experiment in terms of fluorescence intensity data determined for the binding of a specific virus to a specific peptide (1: SEQ ID NO:28, 2: SEQ ID NO:24, 3: SEQ ID NO:32, 4: SEQ ID NO:33, 5: SEQ ID NO:97, 6: SEQ ID NO:98, 7: SEQ ID NO:34, 8: buffer control). It was found that the individual peptides can differ significantly in their binding strength to a specific virus strain or subtype. For example, the binding of H3N2 (X31) increased steadily from peptide with SEQ ID NO:32 to SEQ ID NO:33 to SEQ ID NO:28 to SEQ ID NO:97. On the other hand, the different peptides exhibited proportionally different binding preferences for certain viruses. For instance, SEQ ID NO:97 has, in descending order, a relative preference for H5N1 over H7N1, H3N2 (X31) and H3N2 (NY), whereas SEQ ID NO:33 has a relative preference for H7N1 over H3N2 (X31), H3N2 (NY) and H5N1. Conversely, SEQ ID NO:34 has a preference for H3N2 (NY) over H5N1, H3N2 (X31) and H7N1.

Figure 10 shows corresponding data clouds in a two-dimensional vector space which illustrate the relationship of the binding of the virus strains H3N2 (1968), H1N1 (2009), H5N1, H7N1, H3N2 (Victoria) and H3N2 (New York) to the peptides with SEQ ID NO: 33 (x-axis) and SEQ ID NO:28 (y-axis). These results show that the correlation of the virus binding to these two peptides is already sufficient to obtain a clear distinction for example between virus types H3N2 (1968), H3N2 (Victoria) and H7N1. On the other hand, the correlation of these two peptides was not yet sufficient to clearly distinguish virus types H3N2 (New York) and H5N1. It is however expectable from the results shown in Figure 9 that the correlation with one or more additional peptides, e.g. SEQ ID NO:97, will already lead to a better differentiation and more accurate determination of these virus types, too.

The above-described examples serve as an illustrative description of the underlying concept of the present invention. The examples are not confined to specific combinations of features. Although a number of features and variations have been described only in connection with one particular example or with individual examples, they can, in each case, be combined with any other feature from other examples and the description. It is also possible for particular variations or individual features shown in the examples to be omitted or added, provided that the general technical teaching of the present invention remains realized.

## Claims

1. A peptide having from 8 to 40 amino acids, comprising a sequence selected from a group consisting of

   Phe-Tyr-$X^3$-Tyr-Asp-Val-$X^6$-$X^7$ (SEQ ID NO:9)
   $X^1$-Tyr-$X^3$-Tyr-Asp-Val-Phe-$X^7$ (SEQ ID NO:10),
   Phe-$X^2$-$X^3$-Tyr-Asp-Val-Phe-$X^7$ (SEQ ID NO:11), and
   Phe-Tyr-$X^3$-Tyr-Asp-$X^5$-Phe-$X^7$ (SEQ ID NO:12),

   wherein $X^1$ to $X^7$ are selected from Ala, Asn, Asp, Arg, Cys, Gln, Glu, Gly, His, Ile, Leu, Lys, Met, Phe, Pro, Ser, Thr, Trp, Tyr and Val,
   and wherein at least one of
   $X^1$ is selected from the group consisting of Leu, His, Ile, Trp, Asp, Glu, Gly, Lys, Asn, Pro and Arg;
   $X^2$ is selected from the group consisting of Leu, Pro, Gln, Arg, Cys, Asp, Glu, Ile, Lys, Met and Ser;
   $X^3$ is selected from the group consisting of Tyr, Gly, Phe, Gly, Gln, Thr, Trp and Leu;
   $X^5$ is selected from the group consisting of Pro, Gln, Asp, Glu, Arg;
   $X^6$ is selected from the group consisting of Ala, Asp, Pro, Cys, Glu, Lys, Gln, Gly and Thr; and
   $X^7$ is selected from the group consisting of Phe, Asn, Cys, Asp, Glu, Met, Pro, Leu, Thr, Trp and Ser.

2. The peptide of any one of the preceding claims, further comprising an N-terminally flanking sequence Ala-Arg-Asp and/or a C-terminally flanking sequence Tyr-Ala-Met-Asp.

3. The peptide of any one of the preceding claims, further comprising an oligo lysine $(Lys)_n$ with $2 \leq n \leq 6$.

4. The peptide of any one of the preceding claims, having less than 30, preferably less than 25, more preferably less than 20, most preferred less than 15 amino acids.

5. The peptide of any one of the preceding claims, wherein at least a part of the peptide is cyclized by forming a ring generated by a covalent bond linking an N-terminal moiety and a C-terminal moiety, an N-terminal moiety and a side chain moiety, a C-terminal moiety and a side chain moiety, or two side chain moieties of the peptide.

6. The peptide of any one of the preceding claims, wherein the peptide comprises at least one D-amino acid.

7. A peptide of any one of the preceding claims or a pharmaceutically acceptable salt thereof for use as a medicament, preferably for use in the prevention and/or treatment of influenza A virus infection.

8. A composition comprising a plurality of peptides according to any one of claims 1 to 6 and a supporting material to which the plurality of peptides is physically and/or chemically bonded.

9. A method for diagnosing influenza A virus infection in a subject, comprising:

   A) using a sample of body material from the subject,
   B) distinguishably contacting the sample with i) at least one peptide according to any one of claims 1 to 6 and ii) a control lacking the at least one peptide,
   C) detecting binding to the at least one peptide and to the control,

   wherein an elevated binding to the at least one peptide in comparison to the control is indicative of influenza A virus infection in the subject.

10. A method for determining the type of an influenza A virus, comprising

   a) providing at least a first and a second peptide according to any one of claims 1 to 6, wherein the first and second peptide have a predetermined sequence differing in at least one amino acid from each other,
   b) distinguishably contacting the first and second peptide with a sample containing influenza A virus material,
   c) detecting binding of the influenza A virus material to the first and second peptide,
   d) correlating the binding to the first peptide with the binding to the second peptide,
   e) matching the correlation with a reference correlation of the binding to the first and second peptide of material of a known type of influenza A virus.

**Patentansprüche**

1. Ein Peptid mit 8 bis 40 Aminosäuren, umfassend eine Sequenz ausgewählt aus der Gruppe bestehend aus

   Phe-Tyr-$X^3$-Tyr-Asp-Val-$X^6$-$X^7$ (SEQ ID NO: 9)
   $X^1$-Tyr-$X^3$-Tyr-Asp-Val-Phe-$X^7$ (SEQ ID NO:10),
   Phe-$X^2$-$X^3$-Tyr-Asp-Val-Phe-$X^7$ (SEQ ID NO:11), und
   Phe-Tyr-$X^3$-Tyr-Asp-$X^5$-Phe-$X^7$ (SEQ ID NO:12),

   wobei $X^1$ bis $X^7$ aus Ala, Asn, Asp, Arg, Cys, Gln, Glu, Gly, His, Ile, Leu, Lys, Met, Phe, Pro, Ser, Thr, Trp, Tyr und Val ausgewählt sind,
   und wobei mindestens eines von
   $X^1$ aus der Gruppe bestehend aus Leu, His, Ile, Trp, Asp, Glu, Gly, Lys, Asn, Pro und Arg;
   $X^2$ aus der Gruppe bestehend aus Leu, Pro, Gln, Arg, Cys, Asp, Glu, Ile, Lys, Met und Ser;
   $X^3$ aus der Gruppe bestehend aus Tyr, Gly, Phe, Gly, Gln, Thr, Trp und Leu;
   $X^5$ aus der Gruppe bestehend aus Pro, Gln, Asp, Glu, Arg;
   $X^6$ aus der Gruppe bestehend aus Ala, Asp, Pro, Cys, Glu, Lys, Gln, Gly und Thr; und
   $X^7$ aus der Gruppe bestehend aus Phe, Asn, Cys, Asp, Glu, Met, Pro, Leu, Thr, Trp und Ser
   ausgewählt ist.

2. Das Peptid nach einem der vorhergehenden Ansprüche, zusätzlich umfassend eine N-terminal flankierende Sequenz Ala-Arg-Asp und/oder eine C-terminal flankierende Sequenz Tyr-Ala-Met-Asp.

3. Das Peptid nach einem der vorhergehenden Ansprüche, zusätzlich umfassend ein Oligo-Lysin $(Lys)_n$ mit $2 \leq n \leq 6$.

4. Das Peptid nach einem der vorhergehenden Ansprüche, wobei das Peptid weniger als 30, bevorzugt weniger als 25, besonders bevorzugt weniger als 20, am meisten bevorzugt weniger als 15 Aminosäuren aufweist.

5. Das Peptid nach einem der vorhergehenden Ansprüche, wobei zumindest ein Teil des Peptids durch Bildung eines Rings zyklisiert vorliegt, welcher durch eine kovalente Bindung, die eine N-terminale Gruppe und eine C-terminale Gruppe, eine N-terminale Gruppe und eine Seitenkettengruppe, eine C-terminale Gruppe und eine Seitenkettengruppe oder zwei Seitenkettengruppen des Peptids verbindet, gebildet wird.

6. Das Peptid nach einem der vorhergehenden Ansprüche, wobei das Peptid wenigstens eine D-Aminosäure umfasst.

7. Ein Peptid nach einem der vorhergehenden Ansprüche oder ein pharmazeutisch akzeptables Salz davon zur Verwendung als Medikament, vorzugsweise zur Verwendung in der Prävention und/oder Behandlung einer Influenza-A-Virusinfektion.

8. Eine Anordnung umfassend eine Vielzahl von Peptiden nach einem der Ansprüche 1 bis 6 und ein Trägermaterial, an das die Vielzahl von Peptiden physikalisch und/oder chemisch gebunden ist.

9. Ein Verfahren zur Diagnose einer Influenza-A-Virusinfektion eines Patienten, umfassend:

A) Bereitstellen einer Probe eines Körpermaterials des Patienten,
B) unterscheidbares Kontaktieren der Probe mit i) mindestens einem Peptid nach einem der Ansprüche 1 bis 6 und ii) einer Kontrolle, in der das mindestens eine Peptid nicht enthalten ist,
C) Nachweis einer Bindung an das mindestens eine Peptid und an die Kontrolle,

wobei eine im Vergleich zur Kontrolle erhöhte Bindung an das mindestens eine Peptid auf eine Influenza-A-Virusinfektion des Patienten hinweist.

10. Ein Verfahren zur Bestimmung des Typs eines Influenza-A-Virus, umfassend

a) Bereitstellen mindestens eines ersten und eines zweiten Peptids nach einem der Ansprüche 1 bis 6, wobei das erste und das zweite Peptid eine vorbestimmte Sequenz aufweisen, die sich in mindestens einer Aminosäure voneinander unterscheiden,
b) unterscheidbares Kontaktieren des ersten und zweiten Peptids mit einer Probe, die Material des Influenza-A-Virus enthält,
c) Nachweisen einer Bindung des Materials des Influenza-A-Virus an das erste und zweite Peptid,
d) Korrelieren der Bindung an das erste Peptid mit der Bindung an das zweite Peptid,
e) Vergleichen der Korrelation mit einer Referenzkorrelation einer Bindung an das erste und zweite Peptid von Material eines bekannten Typs von Influenza-A-Virus.

## Revendications

1. Peptide ayant de 8 à 40 acides aminés, comprenant une séquence choisie dans un groupe constitué par

Phe-Tyr-$X^3$-Tyr-Asp-Val-$X^6$-$X^7$ (SEQ ID N° : 9)
$X^1$-Tyr-$X^3$-Tyr-Asp-Val-Phe-$X^7$ (SEQ ID N° : 10),
Phe-$X^2$-$X^3$-Tyr-Asp-Val-Phe-$X^7$ (SEQ ID N° : 11), et
Phe-Tyr-$X^3$-Tyr-Asp-$X^5$-Phe-$X^7$ (SEQ ID N° : 12),

dans lequel $X^1$ à $X^7$ sont choisis parmi Ala, Asn, Asp, Arg, Cys, Gln, Glu, Gly, His, Ile, Leu, Lys, Met, Phe, Pro, Ser, Thr, Trp, Tyr et Val,
et dans lequel au moins l'un de
$X^1$ est choisi dans le groupe constitué par Leu, His, Ile, Trp, Asp, Glu, Gly, Lys, Asn, Pro et Arg ;
$X^2$ est choisi dans le groupe constitué par Leu, Pro, Gln, Arg, Cys, Asp, Glu, Ile, Lys, Met et Ser ;
$X^3$ est choisi dans le groupe constitué par Tyr, Gly, Phe, Gly, Gin, Thr, Trp et Leu ;
$X^5$ est choisi dans le groupe constitué par Pro, Gln, Asp, Glu, Arg ;
$X^6$ est choisi dans le groupe constitué par Ala, Asp, Pro, Cys, Glu, Lys, Gln, Gly et Thr ; et
$X^7$ est choisi dans le groupe constitué par Phe, Asn, Cys, Asp, Glu, Met, Pro, Leu, Thr, Trp et Ser.

2. Peptide selon l'une quelconque des revendications précédentes, comprenant en outre une séquence flanquante N-terminale Ala-Arg-Asp et/ou une séquence flanquante C-terminale Tyr-Ala-Met-Asp.

**3.** Peptide selon l'une quelconque des revendications précédentes, comprenant en outre une oligo lysine (Lys)$_n$ avec $2 \leq n \leq 6$.

**4.** Peptide selon l'une quelconque des revendications précédentes, comportant moins de 30, de préférence moins de 25, plus préférablement moins de 20, mieux encore moins de 15 acides aminés.

**5.** Peptide selon l'une quelconque des revendications précédentes, dans lequel au moins une partie du peptide est cyclisée par la formation d'un cycle généré par une liaison covalente reliant un fragment N-terminal et un fragment C-terminal, un fragment N-terminal et un fragment de chaîne latérale, un fragment C-terminal et un fragment de chaîne latérale, ou deux fragments de chaîne latérale du peptide.

**6.** Peptide selon l'une quelconque des revendications précédentes, dans lequel le peptide comprend au moins un D-aminoacide.

**7.** Peptide selon l'une quelconque des revendications précédentes ou un sel pharmaceutiquement acceptable de celui-ci pour une utilisation comme médicament, de préférence pour une utilisation dans la prévention et/ou le traitement d'une infection par le virus de la grippe A.

**8.** Composition comprenant une pluralité de peptides selon l'une quelconque des revendications 1 à 6 et un matériau de support auquel la pluralité de peptides sont physiquement et/ou chimiquement liés.

**9.** Procédé de diagnostic d'une infection par le virus de la grippe A chez un sujet, comprenant :

A) l'utilisation d'un échantillon de matière corporelle du sujet,
B) la mise en contact distincte de l'échantillon avec i) au moins un peptide selon l'une quelconque des revendications 1 à 6 et ii) un témoin dépourvu de l'au moins un peptide,
C) la détection de la liaison à l'au moins un peptide et au témoin,

dans lequel une liaison élevée à au moins un peptide par rapport au témoin indique une infection par le virus de la grippe A chez le sujet.

**10.** Procédé de détermination du type d'un virus de la grippe A, comprenant

a) la fourniture d'au moins un premier et un second peptides selon l'une quelconque des revendications 1 à 6, dans lequel les premier et second peptides comportent une séquence prédéfinie différant d'au moins un acide aminé l'un de l'autre,
b) la mise en contact distincte des premier et second peptides avec un échantillon contenant du matériel viral de la grippe A,
c) la détection de la liaison du matériel viral de la grippe A aux premier et second peptides,
d) la corrélation de la liaison au premier peptide avec la liaison au second peptide,
e) la mise en correspondance de la corrélation avec une corrélation de référence de la liaison au premier et au second peptides de matériel d'un type connu de virus de la grippe A.

|   | F | Y | D | Y | D | V | F | Y |
|---|---|---|---|---|---|---|---|---|
| A | 7 | 4 | 19 | 22 | -3 | 18 | 4 | -10 |
| C | 27 | 27 | 40 | 23 | -5 | 49 | 41 | 11 |
| D | -7 | 25 | 22 | 41 | 22 | 101 | 162 | 71 |
| E | 6 | 22 | 49 | -2 | 5 | 22 | -3 | 9 |
| F | 22 | 13 | 86 | 5 | -24 | -2 | 22 | -8 |
| G | 38 | 16 | 76 | 16 | -19 | 11 | 42 | -11 |
| H | 35 | 28 | 45 | 17 | -1 | -9 | -11 | -11 |
| I | 34 | 31 | 45 | 47 | -30 | 20 | -7 | -13 |
| K | 32 | 40 | 57 | 12 | -29 | 8 | 10 | 16 |
| L | 33 | 32 | 57 | 7 | -22 | -10 | -6 | 8 |
| M | 32 | 17 | 61 | 6 | -6 | -9 | -4 | 24 |
| N | 42 | 21 | 36 | -26 | -29 | 10 | 14 | 53 |
| P | 18 | 46 | 77 | 5 | -9 | 31 | 20 | 49 |
| Q | 29 | 48 | 52 | -8 | 8 | 15 | -1 | 4 |
| R | 9 | 40 | 47 | 15 | -2 | 19 | -12 | 3 |
| S | 13 | 24 | 44 | -30 | -1 | 11 | 24 | 41 |
| T | 19 | 26 | 56 | -32 | -31 | -12 | 39 | 22 |
| V | 1 | 27 | 26 | 26 | -4 | 22 | 6 | 33 |
| W | 17 | 32 | 48 | -35 | -12 | 0 | 13 | 35 |
| Y | 13 | 22 | 39 | 22 | -15 | 7 | 11 | 22 |

Figure 1

Figure 2

Figure 3

Figure 4

Figure 5

Figure 6

Figure 7

Figure 8

Figure 9

Figure 10

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- EP 14193922

### Non-patent literature cited in the description

- **RAJIK et al.** *Int. J. Biol. Sci.,* 2009, vol. 5, 543-548
- **MATSUBARA et al.** *J. Med. Chem.,* 2009, vol. 52, 4247-4256
- **MATSUBARA et al.** *J. Med. Chem.,* 2010, vol. 53, 4441-4449
- **WU et al.** *PloS One,* 2011, vol. 6, e23058
- **LI et al.** *Peptides,* 2011, vol. 32, 1518-1525
- **LOPEZ-MARTINEZ et al.** *Plos One,* 2013, vol. 8, e76876
- **THOMPSON et al.** *Nucleic Acid Res.,* 1994, vol. 22, 4673-4680
- **ALTSCHUL et al.** *Nucleic Acids Res.,* 1997, vol. 25, 3389-3402
- **BERG et al.** Biochemistry. W. H. Freeman and Company, 2002
- **G. T. HERMANSON.** Bioconjugate Techniques. Academic Press, 2013
- Fmoc Solid phase peptide synthesis: A practical approach. Oxford University Press, 2000
- **FLEURY et al.** *Nat. Struct. Biol.,* 1989, vol. 5, 119-123
- **PAPP et al.** *ChemBioChem,* 2011, vol. 12, 887-895